# EUROPEAN PATENT APPLICATION

(11) **EP 4 817 607 A1**
(43) Date of publication of application: **07.10.2026**
(21) Application number: 25168514.5
(22) Date of filing: 04.04.2025
(51) Int. Cl.: A61K 31/192, A61K 35/66, A61K 45/06, A61P 37/06, G01N 33/48

(54) **DESAMINOTYROSINE (DAT) FOR USE IN PREVENTING OR TREATING GRAFT-VERSUS-HOST DISEASE**

(71) Applicant: Universität Regensburg, in Vertretung des Freistaats Bayern, 93053 Regensburg (DE); Universitätsklinikum Regensburg, 93053 Regensburg (DE)
(72) Inventor: The designation of the inventor has not yet been filed
(74) Representative: Engelhard, Markus

(57) **Abstract**

The present invention relates to desaminotyrosine (DAT) for use in preventing or treating graft-versus-host disease in a patient. The present invention further relates to DAT for use as an adjuvant in an immunotherapy, preferably cancer immunotherapy, of a patient. Furthermore, the present invention relates to a method for predicting an outcome of an immunotherapy.

## Description

### FIELD OF THE INVENTION

The present invention relates to desaminotyrosine (DAT) for use in preventing or treating graft-versus-host disease in a patient. The present invention further relates to DAT for use as an adjuvant in an immunotherapy, preferably cancer immunotherapy, of a patient. Furthermore, the present invention relates to a method for predicting an outcome of an immunotherapy.

### BACKGROUND OF THE INVENTION

Immunotherapies have become very important in the prevention and treatment of cancers. However, the efficiency of immunotherapies is still limited. For example, allogeneic hematopoietic stem cell transplantation (allo-HSCT) is a curative therapy for many otherwise incurable hematological cancers. Yet, its success is limited by the severe morbidity and mortality associated with graft-versus-host disease (GvHD), characterized by an allogeneic immune reaction of donor T cells directed against the recipient's organs, especially the skin, intestine, and liver. However, donor T cells are critical in eliminating residual malignant cells (graft-versus-leukemia (GvL)). Cancer relapse is the primary cause of death in patients receiving immunotherapies such as allo-HSCT, followed by GvHD. Immunosuppressants, corticosteroids, and JAK 1/2 inhibitors are used to treat acute GvHD, but these drugs dampen critical GvL responses, increasing the risk of relapse. Thus, there is a need for enhancing immunotherapies. For example, providing treatments that prevent deleterious GvHD while retaining GvL responses remains an unmet need. Particularly, there is the need for means that allow to prevent or treat GvHD, such as GvHD in patients receiving an allogeneic stem cell transplantation. Furthermore, there is the need for adjuvants for immunotherapies. Moreover, there is the need for predicting the outcome of immunotherapies.

Studies have shown that changes in the intestinal microbiome, including the loss of diversity and expansion of disease-related taxa, are common in patients receiving immunotherapies such as allo-HSCT. These GvHD-related microbial signatures have been attributed to broad-spectrum antibiotics and radio-/chemotherapy-based conditioning regimes but may also be influenced by malnutrition or medication. There remains the need to ameliorate the negative effects associated with the changes in the intestinal microbiome, particularly to ameliorate dysbiosis in cancer patients receiving antibiotic treatments. Furthermore, there remains the need for enhancing immunotherapies in patients receiving antibiotic treatments.

### SUMMARY OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In a first aspect, the present invention relates to desaminotyrosine (DAT) for use in preventing or treating graft-versus-host disease (GvHD) in a patient; preferably in preventing or treating a GvHD in a patient receiving an allogeneic stem cell transplantation.

In one embodiment, DAT is administered to said patient in combination with at least one further microbial metabolite; wherein, optionally, said at least one further microbial metabolite is selected from indole-3-carboxaldehyde, butyric acid, and a combination thereof.

In one embodiment, DAT is administered to said patient by administering a DAT-producing microorganism to said patient; preferably a DAT-producing microorganism selected from *Acinetobacter* sp., *Bacteroides* sp., *Bifidobacterium* sp., *Clostridium* sp., *Enterococcus* sp., *Escherichia* sp., *Eubacterium* sp., *Flavonifractor* sp., *Klebsiella* sp., *Lactiplantibacillus* sp., *Lactobacillus* sp., *Pseudomonas* sp., and *Staphylococcus* sp., such as a DAT-producing microorganism selected from *Acinetobacter baumannii, Bacteroides distasonis, Bacteroides fragilis, Bacteroides thetaiotaomicron, Bifidobacterium Bifidum, Clostridium botulinium, Clostridium sporogenes, Enterococcus faecalis, Enterococcus faecium, Escherichia coli, Eubacterium lentum, Eubacterium ramulus, Flavonifractor plautii, Lactiplantibacillus pentosus, Lactobacillus fermentum,* and *Pseudomonas aeruginosa;* more preferably *Flavonifractor plautii;* even more preferably *Flavonifractor plautii* DSM 6740 or *Flavonifractor plautii* DSM 26117.

In one embodiment, DAT is administered in combination with at least one microorganism; preferably in combination with at least one microorganism selected from *Acinetobacter* sp., *Akkermansia* sp., *Bacteroides* sp., *Bifidobacterium* sp., *Clostridium* sp., *Enterococcus* sp., *Escherichia* sp., *Eubacterium* sp., *Faecalibacterium* sp., *Flavonifractor* sp., *Klebsiella* sp., *Lactiplantibacillus* sp., *Lactobacillus* sp., *Pseudomonas* sp., *Staphylococcus* sp., and combinations thereof,
such as in combination with at least one microorganism selected from *Acinetobacter baumannii, Akkermansia muciniphila, Bacteroides distasonis, Bacteroides fragilis, Bacteroides thetaiotaomicron, Bifidobacterium Bifidum, Clostridium botulinium, Clostridium innocuum, Clostridium sporogenes, Enterococcus faecalis, Enterococcus faecium, Escherichia coli, Eubacterium lentum, Eubacterium ramulus, Flavonifractor plautii, Lactiplantibacillus pentosus, Lactobacillus fermentum, Pseudomonas aeruginosa,* and combinations thereof; more preferably in combination with *Flavonifractor plautii;* even more preferably in combination with *Flavonifractor plautii* DSM 6740 or *Flavonifractor plautii* DSM 26117;
wherein, preferably, said at least one microorganism comprises at least one microorganism producing DAT and optionally producing at least one further microbial metabolite.

In one embodiment, DAT is administered to said patient in the form of a powder, a solution, a suspension, an emulsion, a capsule, a tablet, and/or granules; and/or
DAT is administered to said patient in the form of a stool transplant.

In one embodiment, said patient is characterized by a stool comprising an amount of 0.005 µMol DAT per gram of dried stool or less; and/or
wherein said patient is characterized by a stool which does not comprise *Flavonifractor* sp., *Lachnospiraceae* sp., and/or *Oscillospiraceae* sp., preferably which does not comprise *Flavonifractor plautii.*

In one embodiment, said patient is a patient receiving an antibiotic treatment.

In one embodiment, said patient is characterized by an intact STING1 gene and/or a normal signaling of a stimulator of interferon genes (STING) pathway.

In one embodiment, DAT is used for promoting a regeneration of an intestinal epithelium and/or for an anti-tumor activity by activating effector T cells, optionally by reducing a ratio of Tregs to effector T cells.

In one embodiment, DAT is administered to said patient in a dose in a range of from 1 to 100 mg/kg, preferably in a dose in a range of from 5 to 50 mg/kg, more preferably in a dose in a range of from 10 to 20 mg/kg.

In a second aspect, the present invention relates to DAT for use as an adjuvant in an immunotherapy, preferably cancer immunotherapy, of a patient; wherein, optionally, said immunotherapy, preferably cancer immunotherapy, comprises an allogeneic stem cell transplantation, a CAR T cell therapy, a T cell receptor therapy, and/or an immune checkpoint inhibiting treatment.

In one embodiment, DAT is administered to said patient in combination with at least one further microbial metabolite; wherein, optionally, said at least one further microbial metabolite is selected from indole-3-carboxaldehyde, butyric acid, and a combination thereof.

In one embodiment, DAT is administered to said patient by administering a DAT-producing microorganism to said patient; preferably a DAT-producing microorganism selected from *Acinetobacter* sp., *Bacteroides* sp., *Bifidobacterium* sp., *Clostridium* sp., *Enterococcus* sp., *Escherichia* sp., *Eubacterium* sp., *Flavonifractor* sp., *Klebsiella* sp., *Lactiplantibacillus* sp., *Lactobacillus* sp., *Pseudomonas* sp., and *Staphylococcus* sp., such as a DAT-producing microorganism selected from *Acinetobacter baumannii, Bacteroides distasonis, Bacteroides fragilis, Bacteroides thetaiotaomicron, Bifidobacterium Bifidum, Clostridium botulinium, Clostridium sporogenes, Enterococcus faecalis, Enterococcus faecium, Escherichia coli, Eubacterium lentum, Eubacterium ramulus, Flavonifractor plautii, Lactiplantibacillus pentosus, Lactobacillus fermentum,* and *Pseudomonas aeruginosa;* more preferably *Flavonifractor plautii;* even more preferably *Flavonifractor plautii* DSM 6740 or *Flavonifractor plautii* DSM 26117.

In one embodiment, DAT is administered in combination with at least one microorganism; preferably in combination with at least one microorganism selected from *Acinetobacter* sp., *Akkermansia* sp., *Bacteroides* sp., *Bifidobacterium* sp., *Clostridium* sp., *Enterococcus* sp., *Escherichia* sp., *Eubacterium* sp., *Faecalibacterium* sp., *Flavonifractor* sp., *Klebsiella* sp., *Lactiplantibacillus* sp., *Lactobacillus* sp., *Pseudomonas* sp., *Staphylococcus* sp., and combinations thereof,
such as in combination with at least one microorganism selected from *Acinetobacter baumannii, Akkermansia muciniphila, Bacteroides distasonis, Bacteroides fragilis, Bacteroides thetaiotaomicron, Bifidobacterium Bifidum, Clostridium botulinium, Clostridium innocuum, Clostridium sporogenes, Enterococcus faecalis, Enterococcus faecium, Escherichia coli, Eubacterium lentum, Eubacterium ramulus, Flavonifractor plautii, Lactiplantibacillus pentosus, Lactobacillus fermentum, Pseudomonas aeruginosa,* and combinations thereof; more preferably in combination with *Flavonifractor plautii;* even more preferably in combination with *Flavonifractor plautii* DSM 6740 or *Flavonifractor plautii* DSM 26117;
wherein, preferably, said at least one microorganism comprises at least one microorganism producing DAT and optionally producing at least one further microbial metabolite.

In one embodiment, DAT is administered to said patient in the form of a powder, a solution, a suspension, an emulsion, a capsule, a tablet, and/or granules; and/or
DAT is administered to said patient in the form of a stool transplant.

In one embodiment, said patient is characterized by a stool comprising an amount of 0.005 µMol DAT per gram of dried stool or less; and/or
wherein said patient is characterized by a stool which does not comprise *Flavonifractor* sp., *Lachnospiraceae* sp., and/or *Oscillospiraceae* sp., preferably which does not comprise *Flavonifractor plautii.*

In one embodiment, said patient is a patient receiving an antibiotic treatment.

In one embodiment, said patient is characterized by an intact STING1 gene and/or a normal signaling of a stimulator of interferon genes (STING) pathway.

In one embodiment, DAT is used for promoting a regeneration of an intestinal epithelium and/or for an anti-tumor activity by activating effector T cells, optionally by reducing a ratio of Tregs to effector T cells.

In one embodiment, DAT is administered to said patient in a dose in a range of from 1 to 100 mg/kg, preferably in a dose in a range of from 5 to 50 mg/kg, more preferably in a dose in a range of from 10 to 20 mg/kg.

In a third aspect, the present invention relates to a method for predicting an outcome of an immunotherapy, preferably cancer immunotherapy, comprising the steps of
a) providing a sample of a patient, preferably a stool sample,
b) determining a level of DAT in said sample and comparing said level of DAT to a control;

wherein an increased level of DAT in said sample of said patient compared to said control is indicative for an improved overall survival and/or a reduced relapse; and/or
wherein a decreased level of DAT in said sample of said patient compared to said control is indicative for a reduced overall survival and/or an increased relapse.

In one embodiment, step b) comprises determining whether said sample of said patient comprises more than 0.005 µMol DAT per gram of dried stool, preferably more than 0.02 µMol DAT per gram of dried stool, more preferably more than 0.12 µMol DAT per gram of dried stool; wherein a presence of more than 0.005 µMol DAT per gram of dried stool, preferably more than 0.02 µMol DAT per gram of dried stool, more preferably more than 0.12 µMol DAT per gram of dried stool in said sample is indicative for an improved overall survival and/or a reduced relapse.

In one embodiment, step b) comprises determining whether said sample of said patient comprises 0.01 µMol DAT per gram of dried stool or less, preferably 0.005 µMol DAT per gram of dried stool or less, more preferably less than 0.005 µMol DAT per gram of dried stool, even more preferably 0.0 µMol DAT per gram of dried stool; wherein a presence of 0.01 µMol DAT per gram of dried stool or less, preferably 0.005 µMol DAT per gram of dried stool or less, more preferably less than 0.005 µMol DAT per gram of dried stool, even more preferably 0.0 µMol DAT per gram of dried stool in said sample is indicative for a reduced overall survival and/or an increased relapse.

In one embodiment, said immunotherapy comprises an allogeneic stem cell transplantation, a CAR T cell therapy, a T cell receptor therapy, and/or an immune checkpoint inhibiting treatment. In this aspect, said patient, said immunotherapy, and said DAT are as defined herein.

In a fourth aspect, the present invention relates to a method of preventing or treating graft-versus-host disease (GvHD) in a patient; preferably a method of preventing or treating a GvHD in a patient receiving an allogeneic stem cell transplantation; comprising administering a therapeutically effective amount of DAT to said patient. In this aspect, said patient, said allogeneic stem cell transplantation, said administering, and said DAT are as defined herein.

In a fifth aspect, the present invention relates to a method of immunotherapy, preferably cancer immunotherapy, of a patient; wherein, optionally, said immunotherapy, preferably cancer immunotherapy, comprises an allogeneic stem cell transplantation, a CAR T cell therapy, a T cell receptor therapy, and/or an immune checkpoint inhibiting treatment; wherein said method comprises administering a therapeutically effective amount of DAT as an adjuvant to said patient. In this aspect, said patient, said allogeneic stem cell transplantation, said immunotherapy, said administering, and said DAT are as defined herein.

In a sixth aspect, the present invention relates to a use of DAT for the manufacture of a medicament for preventing or treating graft-versus-host disease (GvHD) in a patient; preferably for preventing or treating a GvHD in a patient receiving an allogeneic stem cell transplantation. In this aspect, said patient, said allogeneic stem cell transplantation, and said DAT are as defined herein.

In a seventh aspect, the present invention relates to a use of DAT for the manufacture of an adjuvant for an immunotherapy, preferably cancer immunotherapy, of a patient; wherein, optionally, said immunotherapy, preferably cancer immunotherapy, comprises an allogeneic stem cell transplantation, a CAR T cell therapy, a T cell receptor therapy, and/or an immune checkpoint inhibiting treatment. In this aspect, said patient, said allogeneic stem cell transplantation, said immunotherapy, and said DAT are as defined herein.

### DETAILED DESCRIPTION

Changes in the intestinal microbiome and microbiota-derived metabolites predict clinical outcomes of immunotherapies, such as after an allogeneic hematopoietic stem cell transplantation (allo-HSCT). The inventors have found that desaminotyrosine (DAT), a product of bacterial flavonoid metabolism, correlates with improved overall survival and reduced relapse rates in immunotherapy patients, such as allo-HSCT patients. Advantageously, treatment with DAT, such as synthetic DAT, prevents graft-versus-host disease by protecting the intestinal barrier and promoting intestinal regeneration and contributes to graft-vs.-leukemia responses. Surprisingly, DAT's beneficial effects on intestinal regeneration remain effective despite broad-spectrum antibiotics-induced dysbiosis, also when administered by fecal microbiota transfer with flavonoid-degrading *F. plautii.* The inventors have found that DAT promotes mTORC1-dependent activation and proliferation of intestinal stem cells, with concomitant engagement of the innate immune receptor STING required to mitigate metabolic stress and maintain an undifferentiated stem cell state independently of type-I interferon responses. Additionally, DAT can skew T cells towards an effector phenotype to modulate graft-versus-leukemia responses. The inventors' data uncover DAT's dual, tissue- and immune-modulating properties and underscore its potential in precision microbiome-based therapies to improve tissue regeneration and minimize immune-mediated side effects.

Herein, the inventors describe an unexpected dual function of DAT which 1) improves tissue regeneration and 2) achieves antitumor responses. DAT's dual function is unique among other reported microbial metabolites in allo-HSCT, suggesting a potential broad applicability as a therapeutic agent to modulate efficacy and toxicity and as a clinical biomarker for predicting outcomes in patients receiving immunotherapies.

In a first aspect, the present invention relates to desaminotyrosine (DAT) for use in preventing or treating graft-versus-host disease (GvHD) in a patient; preferably in preventing or treating a GvHD in a patient receiving an allogeneic stem cell transplantation. The term "patient", as used herein in the context of any aspect of the invention, relates to a human or an animal, preferably a human. The terms "desaminotyrosine" and "DAT", as used herein in the context of any aspect of the invention, may be used interchangeably with the term "3-(4-hydroxyphenyl)propanoic acid". DAT is, for example, a product of bacterial flavonoid metabolism. Said DAT, e.g. said DAT for use according to the first or second aspect of the invention, may be administered to said patient in the form of synthetic DAT, in the form of DAT obtained from a DAT-producing microorganism such as in the form of a DAT extract or in the form of cell culture medium in which a DAT-producing microorganism has been cultivated, and/or by administering a DAT-producing microorganism to said patient.

In one embodiment of any aspect of the invention, DAT is administered to said patient by administering a DAT-producing microorganism, particularly a DAT-producing bacterium, to said patient. In a preferred embodiment of any aspect of the invention, the DAT-producing microorganism is selected from *Acinetobacter* sp., *Bacteroides* sp., *Bifidobacterium* sp., *Clostridium* sp., *Enterococcus* sp., *Escherichia* sp., *Eubacterium* sp., *Flavonifractor* sp., *Klebsiella* sp., *Lactiplantibacillus* sp., *Lactobacillus* sp., *Pseudomonas* sp., and *Staphylococcus* sp. In one embodiment of any aspect of the invention, DAT is administered to said patient by administering a DAT-producing microorganism selected from *Acinetobacter baumannii, Bacteroides distasonis, Bacteroides fragilis, Bacteroides thetaiotaomicron, Bifidobacterium Bifidum, Clostridium botulinium, Clostridium sporogenes, Enterococcus faecalis, Enterococcusfaecium, Escherichia coli, Eubacterium lentum, Eubacterium ramulus, Flavonifractor plautii, Lactiplantibacillus pentosus, Lactobacillus fermentum,* and *Pseudomonas aeruginosa.* In a more preferred embodiment of any aspect of the invention, the DAT-producing microorganism is *Flavonifractor plautii.* In an even more preferred embodiment of any aspect of the invention, the DAT-producing microorganism is *Flavonifractor plautii* DSM 6740 or *Flavonifractor plautii* DSM 26117.

In one embodiment of any aspect of the invention, DAT is administered to said patient in combination with at least one further microbial metabolite. For example, said at least one further microbial metabolite may be selected from indole-3-carboxaldehyde, butyric acid, and a combination thereof. For example, DAT may be administered to said patient in the form of said DAT-producing microorganism which optionally further produces said at least one further microbial metabolite, such as indole-3-carboxaldehyde and/or butyric acid, for example in the form of *Flavonifractor plautii.*

In one embodiment of any aspect of the invention, DAT is administered to said patient in combination with at least one microorganism. For example, DAT is administered to said patient in the form of synthetic DAT, in the form of DAT obtained from a DAT-producing microorganism such as in the form of a DAT extract or in the form of cell culture medium in which a DAT-producing microorganism has been cultivated, and/or by administering a DAT-producing microorganism to said patient, and is administered in combination with said at least one microorganism. Said at least one microorganism may be a DAT-producing microorganism, e.g. if said DAT is administered to said patient in the form of synthetic DAT or in the form of DAT obtained from a DAT-producing microorganism, or said at least one microorganism may be a microorganism which does not produce DAT, such as if DAT is administered to said patient in the form of synthetic DAT, in the form of DAT obtained from a DAT-producing microorganism, and/or by administering a DAT-producing microorganism to said patient. In one embodiment of any aspect of the invention, said at least one microorganism is selected from *Acinetobacter* sp., *Akkermansia* sp., *Bacteroides* sp., *Bifidobacterium* sp., *Clostridium* sp., *Enterococcus* sp., *Escherichia* sp., *Eubacterium* sp., *Faecalibacterium* sp., *Flavonifractor* sp., *Klebsiella* sp., *Lactiplantibacillus* sp., *Lactobacillus* sp., *Pseudomonas* sp., *Staphylococcus* sp., and combinations thereof. In a preferred embodiment of any aspect of the invention, said at least one microorganism is selected from *Acinetobacter baumannii, Akkermansia muciniphila, Bacteroides distasonis, Bacteroides fragilis, Bacteroides thetaiotaomicron, Bifidobacterium Bifidum, Clostridium botulinium, Clostridium innocuum, Clostridium sporogenes, Enterococcus faecalis, Enterococcus faecium, Escherichia coli, Eubacterium lentum, Eubacterium ramulus, Flavonifractor plautii, Lactiplantibacillus pentosus, Lactobacillus fermentum, Pseudomonas aeruginosa,* and combinations thereof. In a more preferred embodiment of any aspect of the invention, said at least one microorganism comprises *Flavonifractor plautii.* In an even more preferred embodiment of any aspect of the invention, said at least one microorganism comprises *Flavonifractor plautii* DSM 6740 and/or *Flavonifractor plautii* DSM 26117. In one embodiment of any aspect of the invention, said at least one microorganism comprises a DAT-producing microorganism and optionally a further microorganism which produces one or more microbial metabolites other than DAT. In a preferred embodiment of any aspect of the invention, said at least one microorganism comprises at least one microorganism producing DAT and optionally producing at least one further microbial metabolite.

In a preferred embodiment of any aspect of the invention, the allogeneic stem cell transplantation is an allogeneic hematopoietic stem cell transplantation (allo-HSCT). The inventors have found that DAT is effective in the prevention and treatment of GvHD in patients receiving an allogeneic stem cell transplantation, such as an allogeneic hematopoietic stem cell transplantation.

In one embodiment of any aspect of the invention, DAT is administered to said patient in the form of a powder, a solution, a suspension, an emulsion, a capsule, a tablet, and/or granules. For example, synthetic DAT, DAT obtained from a DAT-producing microorganism, and/or a DAT-producing microorganism may be administered to said patient in the form of a powder, a solution, a suspension, an emulsion, a capsule, a tablet, and/or granules. In one embodiment of any aspect of the invention, DAT is formulated as a powder, a solution, a suspension, an emulsion, a capsule, a tablet, and/or granules. For example, synthetic DAT, DAT obtained from a DAT-producing microorganism, and/or a DAT-producing microorganism may be formulated as a powder, a solution, a suspension, an emulsion, a capsule, a tablet, and/or granules. In one embodiment of any aspect of the invention, DAT is administered to said patient in the form of a stool transplant. In one embodiment of any aspect of the invention, said stool transplant comprises a DAT-producing microorganism and/or DAT obtained from a DAT-producing microorganism. In one embodiment of any aspect of the invention, DAT is administered in the form of a stool transplant; wherein, optionally, said stool transplant is formulated as a powder, a solution, a suspension, an emulsion, a capsule, a tablet, and/or granules.

In one embodiment of any aspect of the invention, said patient is characterized by a stool comprising an amount of 0.005 µMol DAT per gram of dried stool or less. The inventors have found that GvHD is efficiently prevented or treated by DAT in patients, such as patients receiving an allogeneic stem cell transplantation, characterized by a stool comprising an amount of 0.005 µMol DAT per gram of dried stool or less. In one embodiment of any aspect of the invention, said dried stool is provided by providing a defined amount of stool, such as 5 ml, and subsequently drying said stool.

In one embodiment of any aspect of the invention, said patient is characterized by a stool which does not comprise *Flavonifractor* sp., *Lachnospiraceae* sp., and/or *Oscillospiraceae* sp., preferably which does not comprise *Flavonifractor plautii.* The inventors have found that GvHD is efficiently prevented or treated by DAT in patients, such as patients receiving an allogeneic stem cell transplantation, characterized by a stool which does not comprise *Flavonifractor* sp., *Lachnospiraceae* sp., and/or *Oscillospiraceae* sp., preferably which does not comprise *Flavonifractor plautii.* The inventors have found that patients which have reduced levels of DAT and/or DAT-producing microorganisms such as *Flavonifractor* sp., *Lachnospiraceae* sp., and/or *Oscillospiraceae* sp., particularly *Flavonifractor plautii,* in their gastrointestinal tract, e.g. as evidenced by their stool, strongly benefit from a treatment with DAT, such as DAT administered in the form of synthetic DAT, in the form of DAT obtained from a microorganism, and/or in the form of a DAT-producing microorganism.

For example, said DAT, e.g. administered in the form of synthetic DAT, in the form of DAT obtained from a microorganism, and/or in the form of a DAT-producing microorganism, may be administered once daily, preferably once daily for at least one week, such as for about one week, about two weeks, about three weeks, or about four weeks. In one embodiment of any aspect of the invention, DAT is administered once daily for at least one week, such as once daily for one to two weeks, once daily for one to three weeks, or once daily for one to four weeks. In one embodiment of any aspect of the invention, DAT is administered, e.g. once daily, for 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 days or more.

In one embodiment of any aspect of the invention, said patient is a patient receiving an antibiotic treatment, such as a treatment with a broad-spectrum antibiotic, e.g. Meropenem. The term "receiving an antibiotic treatment", as used herein, may relate to simultaneously receiving an antibiotic treatment, such as an antibiotic treatment that is administered to said patient at a same day as DAT for at least one day, and/or may relate to an antibiotic treatment which has ended at most 4 weeks, preferably at most 3 weeks, more preferably at most 1 week, even more preferably at most 1 day, before an administration of DAT to said patient, and/or to an antibiotic treatment which is about to start at most 4 weeks, preferably at most 3 weeks, more preferably at most 1 week, even more preferably at most 1 day, after an administration of DAT to said patient. In a preferred embodiment of any aspect of the invention, said patient receiving an antibiotic treatment is a patient simultaneously receiving an antibiotic treatment, particularly simultaneously receiving said DAT and said antibiotic treatment, and/or is a patient having received an antibiotic treatment, wherein said antibiotic treatment has ended at most 4 weeks, preferably at most 3 weeks, more preferably at most 1 week, such as 1 day, before an administration of said DAT to said patient. For example, DAT may be administered to said patient before, during, and/or after an antibiotic treatment; preferably during and/or after an antibiotic treatment, such as during an antibiotic treatment, after an antibiotic treatment, or during and after an antibiotic treatment. In one embodiment of any aspect of the invention, said patient is a patient suffering from or being at risk of acquiring a dysbiosis, such as a dysbiosis induced by an antibiotic treatment. The term "dysbiosis", as used herein in the context of a patient of any aspect of the invention, preferably relates to an imbalance of the patient's microbiome, particularly an imbalance of the patient's microbiome induced by an antibiotic treatment, for example a microbiome having a reduced amount of DAT-producing microorganisms. In one embodiment of any aspect of the invention, said patient is characterized by a microbiome, particularly a gastrointestinal microbiome, having a reduced amount of DAT-producing microorganisms. In one embodiment of any aspect of the invention, said patient is a patient receiving an antibiotic treatment and an allogeneic stem cell transplantation. In one embodiment of any aspect of the invention, said DAT for use in preventing or treating GvHD in a patient is for use in a patient receiving an antibiotic treatment and an allogeneic stem cell transplantation. In one embodiment of any aspect of the invention, said DAT for use as an adjuvant in an immunotherapy of a patient is for use in a patient receiving an antibiotic treatment and an allogeneic stem cell transplantation.

In one embodiment of any aspect of the invention, said patient is characterized by an intact STING1 gene and/or a normal signaling of a stimulator of interferon genes (STING) pathway. In a preferred embodiment of any aspect of the invention, said patient is characterized by an intact STING1 gene. The term "normal signaling", as used herein, relates to a non-aberrant signaling, particularly to a signaling which corresponds to a reference value and/or reference sample, wherein said reference value and/or sample is preferably indicative of an expected signaling in healthy individuals. For example, normal STING pathway signaling is signaling that is neither overactive nor underactive STING pathway signaling compared to a healthy individual or a pool of healthy individuals. The term "intact", as used herein in the context of the STING1 gene, is meant the be understood as preferably referring to a gene which does not comprise any mutations or which only comprises silent mutations.

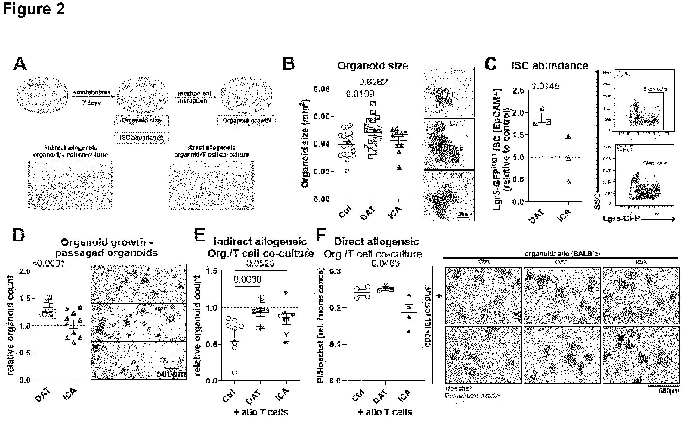

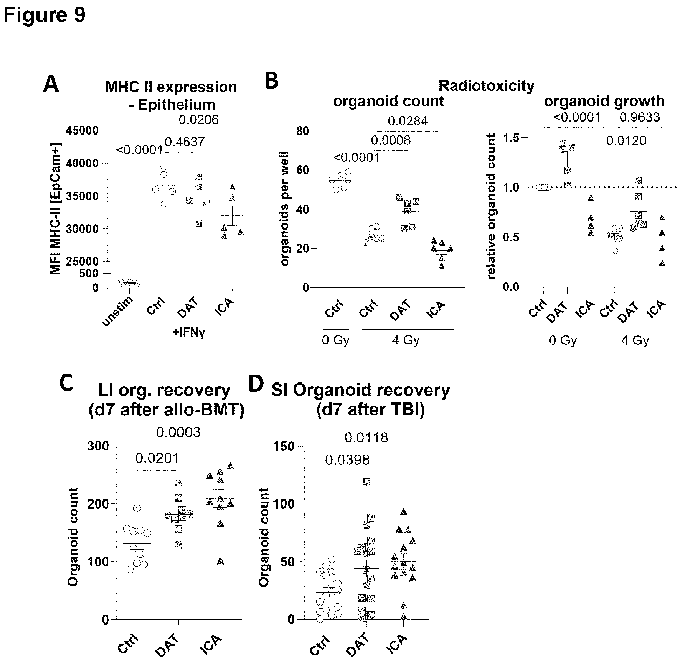

In one embodiment of any aspect of the invention, DAT is used for promoting a regeneration of an intestinal epithelium. The inventors have found that, advantageously, DAT promotes the regeneration of intestinal epithelium. For example, DAT for use in preventing or treating GvHD in a patient promotes a regeneration of an intestinal epithelium of said patient. For example, DAT for use as an adjuvant in an immunotherapy of a patient promotes a regeneration of an intestinal epithelium of said patient. In one embodiment, DAT for use in preventing or treating GvHD in a patient comprises promoting a regeneration of an intestinal epithelium. In one embodiment, DAT for use in preventing or treating GvHD in a patient is used for promoting a regeneration of an intestinal epithelium. The inventors have found that, surprisingly, DAT effectively promotes a regeneration of an intestinal epithelium, e.g. as indicated by organoid regeneration, while ICA was much less effective (Fig. 2E). Furthermore, the inventors have found that, advantageously, a treatment with DAT effectively promotes a regeneration of an intestinal epithelium, e.g. as indicated by an improvement in the number of viable organoids and organoid expansion following irradiation which is not observed for ICA (Fig. 9B). In one embodiment of any aspect of the invention, said patient is a patient receiving a radiotherapy. The inventors have found that, surprisingly, DAT promotes a regeneration of the intestinal epithelium and protects it from radiation.

In one embodiment of any aspect of the invention, DAT is used for an anti-tumor activity by activating effector T cells, optionally by reducing a ratio of Tregs to effector T cells. The inventors have found that, advantageously, DAT has an anti-tumor activity by activating effector T cells, e.g. by reducing a ratio of Tregs to effector T cells. For example, DAT for use in preventing or treating GvHD in a patient additionally has an anti-tumor activity by activating effector T cells, e.g. by reducing a ratio of Tregs to effector T cells. For example, DAT for use as an adjuvant in an immunotherapy of a patient has an anti-tumor activity by activating effector T cells, e.g. by reducing a ratio of Tregs to effector T cells, in said patient. In one embodiment, DAT for use as an adjuvant in an immunotherapy of a patient comprises an anti-tumor activity by activating effector T cells, e.g. by reducing a ratio of Tregs to effector T cells, in said patient. In one embodiment, DAT for use as an adjuvant in an immunotherapy of a patient is used for an anti-tumor activity by activating effector T cells, optionally by reducing a ratio of Tregs to effector T cells.

In one embodiment of any aspect of the invention, DAT is administered to said patient in a dose in a range of from 1 to 100 mg/kg, preferably in a dose in a range of from 5 to 50 mg/kg, more preferably in a dose in a range of from 10 to 20 mg/kg. The unit "mg/kg", as used herein in the context of a dose of DAT, relates to mg of DAT per kg body weight of a patient. In a preferred embodiment of any aspect of the invention, DAT is administered to said patient in a dose which is < 275 mg/kg, preferably < 200 mg/kg.

For example, said DAT may be administered, e.g. in the form of synthetic DAT, in the form of DAT obtained from a microorganism, and/or in the form of a DAT-producing microorganism, to said patient once, once daily, twice daily, once every other day, or once weekly, preferably once daily.

In a second aspect, the present invention relates to DAT for use as an adjuvant in an immunotherapy, preferably cancer immunotherapy, of a patient; wherein, optionally, said immunotherapy, preferably cancer immunotherapy, comprises an allogeneic stem cell transplantation, a CAR T cell therapy, a T cell receptor therapy, and/or an immune checkpoint inhibiting treatment. In one embodiment of any aspect of the invention, said immunotherapy is a cancer immunotherapy, preferably selected from an allogeneic stem cell transplantation, a CAR T cell therapy, a T cell receptor therapy, and an immune checkpoint inhibiting treatment. For example, the term "adjuvant", as used herein, relates to an enhancer of an immunotherapy, for example an enhancer of the patient's immune response to cancer cells. The term "immunotherapy", as used herein in the context of any aspect of the invention, preferably relates to a cancer immunotherapy, more preferably to an allogeneic stem cell transplantation, a CAR T cell therapy, a T cell receptor therapy, and/or an immune checkpoint inhibiting treatment. In one embodiment of any aspect of the invention, said immunotherapy comprises or is an allogeneic stem cell transplantation, a CAR T cell therapy, a T cell receptor therapy, or an immune checkpoint inhibiting treatment. In one embodiment of the second aspect of the invention, said immunotherapy is not an immune checkpoint inhibiting treatment, wherein, preferably, said immunotherapy is selected from an allogeneic stem cell transplantation, a CAR T cell therapy, and a T cell receptor therapy. In one embodiment, said DAT for use as an adjuvant in an immunotherapy of a patient is for use in an immunotherapy of a patient receiving an antibiotic treatment, such as a treatment with a broad-spectrum antibiotic, e.g. Meropenem. The inventors have found that, advantageously, DAT significantly reduces tumor burden and prolongs overall survival. Furthermore, the inventors have found that, surprisingly, DAT exerts dual roles in allogeneic stem cell transplantations such as in allo-HSCT, particularly protecting from GvHD while contributing to GvL. In one embodiment, said DAT for use in preventing or treating GvHD in a patient further comprises said DAT for use as an adjuvant in an immunotherapy of a patient, preferably in an allogeneic stem cell transplantation of said patient. In one embodiment, said DAT for use as an adjuvant in an immunotherapy of a patient, preferably in an allogeneic stem cell transplantation of said patient, further comprises said DAT for use in preventing or treating GvHD in a patient. In one embodiment, said DAT for use of the invention is for use in preventing or treating GvHD in a patient and for use as an adjuvant in an immunotherapy, preferably allogeneic stem cell transplantation, of said patient. In one embodiment, said DAT for use as an adjuvant in an immunotherapy, such as in an allogeneic stem cell transplantation, further comprises preventing or treating GvHD in said patient.

In an eighth aspect, the present invention relates to a method for predicting an outcome of an immunotherapy, preferably cancer immunotherapy, comprising the step of
determining a level of DAT in a sample, preferably stool sample, of a patient, and comparing said level of DAT to a control;
wherein an increased level of DAT in said sample of said patient compared to said control is indicative for an improved overall survival and/or a reduced relapse; and/or
wherein a decreased level of DAT in said sample of said patient compared to said control is indicative for a reduced overall survival and/or an increased relapse. Said method, said determining, said patient, and/or said immunotherapy are as defined herein, particularly as defined herein in the context of any other aspect of the invention.

In a third aspect, the present invention relates to a method for predicting an outcome of an immunotherapy, preferably cancer immunotherapy, comprising the steps of
a) providing a sample of a patient, preferably a stool sample,
b) determining a level of DAT in said sample and comparing said level of DAT to a control;

wherein an increased level of DAT in said sample of said patient compared to said control is indicative for an improved overall survival and/or a reduced relapse; and/or
wherein a decreased level of DAT in said sample of said patient compared to said control is indicative for a reduced overall survival and/or an increased relapse.

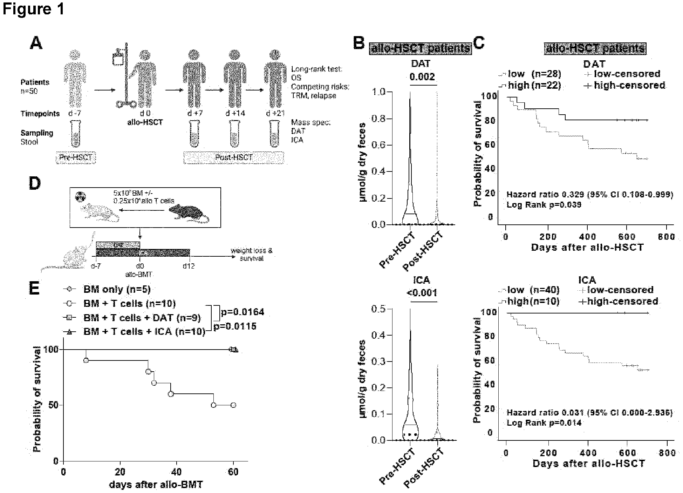

The inventors have found that, advantageously, high levels of DAT correlate with improved overall survival, such as improved two-year overall survival (Fig. 1 C).

In one embodiment of the third or eighth aspect, the control is a reference value and/or reference sample, such as of a healthy individual, of a pool of healthy individuals, of an individual having a good prognosis, or of a pool of individuals having a good prognosis. For example, said control may be a reference value, such as 0.0 µMol DAT per gram of dried stool, 0.005 µMol DAT per gram of dried stool, 0.02 µMol DAT per gram of dried stool, or 0.12 µMol DAT per gram of dried stool, preferably 0.005 µMol DAT per gram of dried stool. In one embodiment of the third or eighth aspect, said control is a reference value of 0.005 µMol DAT per gram of dried stool. In one embodiment of the third or eighth aspect, an increased level of DAT in said sample of said patient compared to a control, said control being a reference value which is 0.005 µMol DAT per gram of dried stool, is indicative for an improved overall survival and/or a reduced relapse. In one embodiment of the third or eighth aspect, a decreased level of DAT in said sample of said patient compared to a control, said control being a reference value which is 0.005 µMol DAT per gram of dried stool, is indicative for a reduced overall survival and/or an increased relapse.

In one embodiment of the third or eighth aspect, an increased level of DAT in said sample of said patient compared to said control is indicative for an improved overall survival and/or a reduced relapse; and a decreased level of DAT compared to said control or a level of DAT which is equal to said control is indicative for a reduced overall survival and/or an increased relapse; wherein, optionally, said control is 0.005 µMol DAT per gram of dried stool.

In one embodiment of the third or eighth aspect, said control is 0.005 µMol DAT per gram of dried stool, 0.02 µMol DAT per gram of dried stool, or 0.12 µMol DAT per gram of dried stool, preferably 0.005 µMol DAT per gram of dried stool, wherein an increased level of DAT in said sample of said patient compared to said control is indicative for an improved overall survival and/or a reduced relapse. In one embodiment of the third or eighth aspect, said control is 0.01 µMol DAT per gram of dried stool, 0.005 µMol DAT per gram of dried stool, or 0.0 µMol DAT per gram of dried stool, preferably 0.01 µMol DAT per gram of dried stool or 0.005 µMol DAT per gram of dried stool, more preferably 0.005 µMol DAT per gram of dried stool, wherein a decreased level of DAT compared to said control or a level of DAT which is equal to said control is indicative for a reduced overall survival and/or an increased relapse.

In one embodiment of the third or eighth aspect, said determining a level of DAT in said sample and comparing said level of DAT to a control comprises comparing said level of DAT to a reference value of 0.005 µMol DAT per gram of dried stool, wherein an increased level of DAT in said sample of said patient compared to said reference value of 0.005 µMol DAT per gram of dried stool is indicative for an improved overall survival and/or a reduced relapse, and wherein a decreased level of DAT compared to said reference value of 0.005 µMol DAT per gram of dried stool or a level of DAT which is equal to said reference value of 0.005 µMol DAT per gram of dried stool is indicative for a reduced overall survival and/or an increased relapse.

In one embodiment of the third or eighth aspect, said method is an in vitro and/or ex vivo method. In one embodiment of the third or eighth aspect, the sample of said patient is a sample non-invasively obtained from said patient. In one embodiment of the third or eighth aspect, said method is a non-invasive method. In one embodiment, step a) of said method is non-invasive. In one embodiment, said providing a sample of a patient is non-invasive. In one embodiment, said providing a sample of a patient comprises or consists of non-invasively obtaining a sample from said patient. For example, said sample of a patient may be a stool sample, a urine sample, or a blood sample such as a serum sample; preferably is a stool sample or a urine sample; more preferably is a stool sample.

In one embodiment of the third or eighth aspect, said determining comprises determining whether said sample of said patient comprises more than 0.005 µMol DAT per gram of dried stool, preferably more than 0.02 µMol DAT per gram of dried stool, more preferably more than 0.12 µMol DAT per gram of dried stool; wherein a presence of more than 0.005 µMol DAT per gram of dried stool, preferably more than 0.02 µMol DAT per gram of dried stool, more preferably more than 0.12 µMol DAT per gram of dried stool in said sample is indicative for an improved overall survival and/or a reduced relapse. In one embodiment of the third or eighth aspect, said determining comprises determining whether said sample of said patient comprises 0.01 µMol DAT per gram of dried stool or less, preferably 0.005 µMol DAT per gram of dried stool or less, more preferably less than 0.005 µMol DAT per gram of dried stool, even more preferably 0.0 µMol DAT per gram of dried stool; wherein a presence of 0.01 µMol DAT per gram of dried stool or less, preferably 0.005 µMol DAT per gram of dried stool or less, more preferably less than 0.005 µMol DAT per gram of dried stool, even more preferably 0.0 µMol DAT per gram of dried stool in said sample is indicative for a reduced overall survival and/or an increased relapse. For example, said determining may comprise determining said DAT using mass spectrometry.

In one embodiment of the third or eighth aspect, said immunotherapy comprises an allogeneic stem cell transplantation such as an allogeneic hematopoietic stem cell transplantation, a CAR T cell therapy, a T cell receptor therapy, and/or an immune checkpoint inhibiting treatment. Said method for predicting an outcome of an immunotherapy according to the third or eighth aspect may be combined with said DAT for use in preventing or treating GvHD in a patient and/or said DAT for use as an adjuvant in an immunotherapy of a patient. For example, said method for predicting an outcome of an immunotherapy of the third or eighth aspect may comprise determining a decreased level of DAT in said sample of said patient compared to said control and subsequently, particularly to enhance the overall survival and/or reduce relapse of said patient, administering DAT for use according to the first or second aspect of the invention to said patient. For example, said method for predicting an outcome of an immunotherapy of the third or eighth aspect may comprise determining a decreased level of DAT in said sample of said patient compared to said control and subsequently administering DAT for use according to the first or second aspect of the invention to said patient for promoting a regeneration of an intestinal epithelium of said patient and/or for an anti-tumor activity by activating effector T cells in said patient.

In a fourth aspect, the present invention relates to a method of preventing or treating graft-versus-host disease (GvHD) in a patient; preferably a method of preventing or treating a GvHD in a patient receiving an allogeneic stem cell transplantation; comprising administering a therapeutically effective amount of DAT to said patient. Said patient, said allogeneic stem cell transplantation, said administering, and said DAT may be as defined herein, particularly as defined herein in the context of any other aspect of the invention. The term "therapeutically effective amount", as used herein in the context of any aspect of the invention, relates to an amount which achieves the desired therapeutic effect, such as the prevention or treatment of GvHD or such as an enhancement of an immunotherapy.

In a fifth aspect, the present invention relates to a method of immunotherapy of a patient; wherein said method comprises administering a therapeutically effective amount of DAT as an adjuvant to said patient. Said immunotherapy, said patient, said therapeutically effective amount, said administering, said adjuvant, and/or said DAT may be as defined herein, particularly as defined herein in the context of any other aspect of the invention.

In a sixth aspect, the present invention relates to a use of DAT for the manufacture of a medicament for preventing or treating graft-versus-host disease (GvHD) in a patient; preferably for preventing or treating a GvHD in a patient receiving an allogeneic stem cell transplantation. Said medicament may be administered to said patient as described herein, e.g. in the context of the first aspect of the invention. For example, said medicament may be formulated as a powder, a solution, a suspension, an emulsion, a capsule, a tablet, and/or granules; and/or a stool transplant. Said patient, said preventing or treating GvHD, said administering, said allogeneic stem cell transplantation, and/or said DAT may be as defined herein, particularly as defined herein in the context of any other aspect of the invention.

In a seventh aspect, the present invention relates to a use of DAT for the manufacture of an adjuvant for an immunotherapy, preferably cancer immunotherapy, of a patient; wherein, optionally, said immunotherapy, preferably cancer immunotherapy, comprises an allogeneic stem cell transplantation, a CAR T cell therapy, a T cell receptor therapy, and/or an immune checkpoint inhibiting treatment. Said medicament may be administered to said patient as described herein, e.g. in the context of the second aspect of the invention. Said adjuvant, said immunotherapy, said patient, said administering, said allogeneic stem cell transplantation, and/or said DAT may be as defined herein, particularly as defined herein in the context of any other aspect of the invention.

The terms "of the [present] invention", "in accordance with the invention", "according to the invention" and the like, as used herein are intended to refer to all aspects and embodiments of the invention described and/or claimed herein. The term "of any aspect of the invention", as used herein, relates to all aspects of the invention, particularly to the first, second, third, fourth, fifth, sixth, seventh, and eighth aspect of the invention. Embodiments described herein with respect to any aspect of the invention are meant to be understood as also being directly and unambiguously disclosed with respect to the other aspects of the invention, for example embodiments described with respect to the first aspect, e.g. embodiments relating to the administration of DAT, are meant to be understood as also being directly and unambiguously disclosed with respect to the other aspects of the invention such as with respect to the second aspect. As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated. The term "about", as used herein in the context of numbers or data, intends to include deviations (±) which usually are to be considered in the respective technical field.

### BRIEF DESCRIPTION OF THE FIGURES

The present invention is now further described by reference to the following figures.

All methods mentioned in the figure descriptions below were carried out as described in detail in the examples.

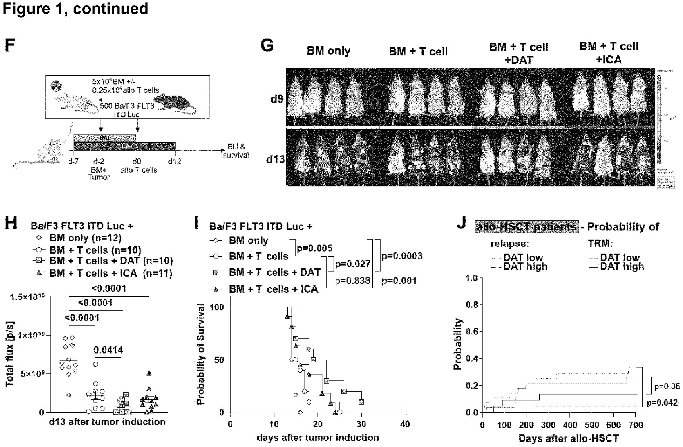

**Fig. 1**: **The microbial metabolite DAT modulates clinical and preclinical GvHD and GvL. (A)** Study overview. **(B)** Stool metabolite concentrations in patients receiving allo-HSCT before (day -7) and after (day +7 to +21) transplantation measured by targeted mass-spectrometry in 50 patients from Munich and Regensburg. Data is presented as violin plot with median as line and quartiles as dotted lines and analyzed by non-parametric paired t-test. **(C)** Kaplan Meier plots of patient's overall survival after allo-HSCT grouped by stool metabolite concentrations of DAT or ICA based on cut-offs (DAT=0.005/µmol/g dry feces; ICA=0.035µmol/g dry feces). Data was analyzed by Log-rank test. **(D)** Experimental design of the murine MHC major mismatch allo-BMT model. **(E)** Survival of mice after allo-BMT. Pooled data from 2 independent experiments. Analyzed by Log-rank test. The inventors have found that, advantageously, DAT is highly useful for preventing or treating GvHD in a patient. **(F)** Experimental design of the GvL model in **G-I. (G)** Representative BLI images and **(H)** quantification of BLI signal on d13 from two independent experiments. P-values were calculated using two-way ANOVA with Dunnett's correction for multiple comparisons or unpaired t-test. The inventors have found that, advantageously, DAT is highly useful for enhancing an immunotherapy of a patient. **(I)** Kaplan Meier plot of survival. Data was analyzed by Log-rank test from 2 independent experiments. The inventors have found that, surprisingly, DAT is more effective in enhancing an immunotherapy than ICA. **(J)** Cumulative incidence of relapse and TRM after 2 years after day +21 in a competing risk analysis, stratified according to DAT high and DAT low. Cumulative incidence functions between groups were tested for equality using Gray's test. If not depicted otherwise, all data are shown as mean ± SEM.

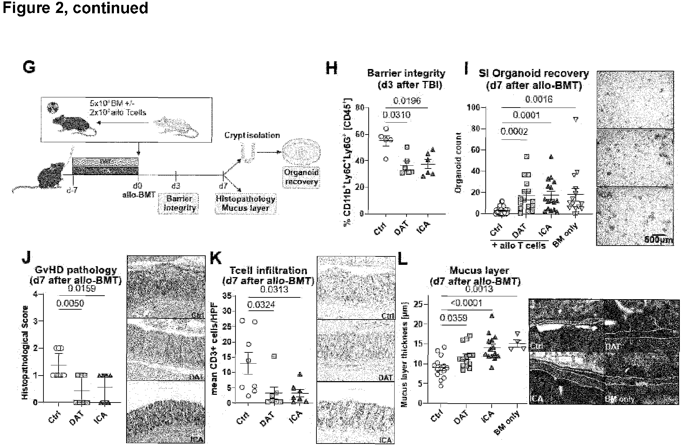

**Fig. 2****: DAT promotes growth and damage protection in murine organoids and in vivo damage models. (A)** Experimental overview. **(B-D)** Murine SI organoids were stimulated with 100 µM DAT, 50 µM ICA or carrier. **(B)** Representative images and quantification of organoid size after 6 days of metabolite treatment. Data pooled from 10 or 20 independent experiments. **(C)** Lgr₅-GFP^{high} ISC abundance assessed by flow cytometry after 6 days of metabolite treatment. Representative FACS plots and abundance of Lgr₅-GFP^{high} intestinal stem cells (ISCs) relative to untreated organoids from 3 independent experiments are shown. **(D)** Number of established organoids after passaging relative to control (indicated by the dotted line). Data was pooled from 10 independent experiments. **(E)** Murine SI organoids were co-cultured with activated splenic T cells in the surrounding media for 4 days without direct interaction and their growth was assessed as described in (D). Data was pooled from 8 independent experiments. **(F)** SI organoids were brought in direct contact with allogeneic intraepithelial T cells. After 48h, cell death was detected by propidium iodide fluorescence and normalized to Hoechst fluorescence. Representative images and PI/Hoechst quantification. Pooled data from 3-4 independent experiments. **(G)** Utilized in *vivo* experimental design. **(H)** Infiltration of CD11b+Ly6G+Ly6C+ granulocytes of total live CD45+ cells in ileum was assessed by flow cytometry on d3 after 9 Gy TBI. Pooled data from 3 (n=5-6 mice/group) independent experiments. **(I)** Count of recovered small intestinal organoids on day 7 after allo-BMT and representative pictures. Pooled data from 6 experiments (n=15-29). **(J)** Histopathology score and **(K)** number of infiltrating T cells in the colon of mice treated as in (Fig. 2G). Pooled data from 3 independent experiments (n=7-8). **(L)** Quantification and representative pictures of the large intestinal mucus layer thickness. Pooled data from 3 independent experiments (n=4-15). P-values were calculated by ordinary one-way ANOVA with Dunnett's correction for multiple comparisons (B, E, F, J, K, L), Kruskal-Wallis test with Dunn's correction for multiple comparisons (H, I) or one sample t-test (C, D). All data are shown as mean ± SEM.

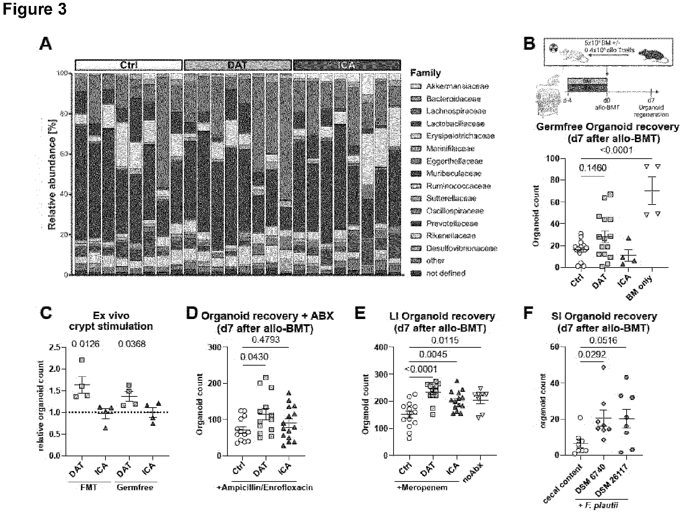

**Fig. 3****: DAT treatment is effective in SPF but not GF mice and remains effective after antibiotic-induced dysbiosis. (A)** Mice were treated with metabolites as described in Fig. 2 G, stool samples (n=8) were collected on do and analyzed by 16SrRNA sequencing. Relative abundance on family level is shown. **(B)** Experimental layout for treatment of germfree mice and organoid regeneration was assessed as described for Fig. 2I but Rock inhibitor was added to the culture medium. Pooled data from 4 experiments (n=4 or 15). **(C)** Small intestinal crypts were isolated from GF BALB/c mice or littermates reconstituted by FMT. Crypts were stimulated directly after initiation of culture and organoid growth was assessed as in Fig. 2D relative to its respective control. Pooled data from 4 independent experiments. **(D)** C57BL/6 mice were continuously treated with Ampicillin/Enrofloxacin added to their drinking water beginning one week before metabolite treatment. Allo-BMT and organoid recovery were performed as in Fig. 2I but in presence of Rock inhibitor. Pooled data from 3 independent experiments (n=13 or 15). **(E)** C57BL/6 mice were treated with meropenem per drinking water for 7 days for induction of dysbiosis before metabolite treatment, allo-BMT with 1.5x10⁶ allogeneic T cells and large intestinal organoid regeneration was performed as outlined in Fig. 9C. Pooled data from 3 independent experiments (n=8-14). **(F)** Mice were treated with ampicillin/enrofloxacin for 5 days beginning on day -14. On day -7 and -5, mice received FMT with cecal content of SPF mice or human or murine *F. plautii* isolates (DSM 6740 or 26117, respectively) followed by allo-BMT and scoring of SI organoid regeneration. P-values were calculated with ordinary one-way ANOVA with Dunnett's correction for multiple comparisons (B, D, E), Kruskal-Wallis test with Dunn's correction for multiple comparisons (F) or one sample t-test (C). All data are shown as mean ± SEM.

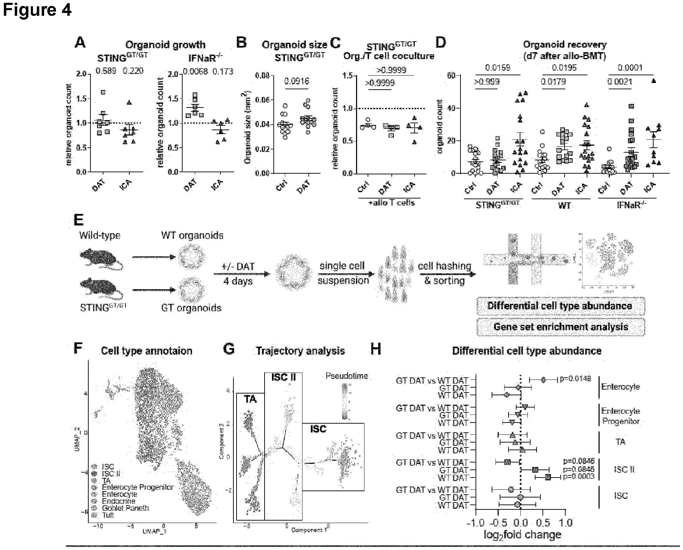

**Fig. 4****: DAT acts on the intestinal stem cell compartment and requires STING but not IFN-I signaling. (A)** The growth of SI organoids derived from STING^{GT}/^{GT} and IFNaR^{-/-}mice was assessed as described in Fig. 2D. Pooled data from 6-7 independent experiments. P-values were calculated by one sample t-test. **(B)** Small intestinal STING^{GT}/^{GT} organoid size was assessed as in Fig. 2B. Pooled data from 13 independent experiments analyzed by unpaired t-test. **(C)** STING-deficient organoids were co-cultured with allogeneic activated T cells as in Fig. 2E. Pooled data from 4 independent experiments analyzed by Kruskal-Wallis test with Dunn's correction for multiple comparisons. **(D)** Organoid recovery was assessed in C57BL/6 WT, STING^{GT}/^{GT} or IFN-I receptor deficient mice that were treated with metabolites and underwent allo-BMT as in Fig. 2I. Pooled data from 4-7 individual experiments (n=15-19 mice/group). P-values were calculated using Kruskal-Wallis test with Dunn's correction for multiple comparisons for depicted genotypes individually. **(E-G)** Murine small intestinal organoids generated from WT of STING^{GT}/^{GT} mice were stimulated with metabolites as before for 4 days and subsequently analyzed by scRNA-seq. Pooled data from 3 independent experiments. **(E)** Experimental layout. **(F)** Plot of single cells in UMAP space from all experimental conditions, colored by final cell type annotation. **(G)** Ordering of the ISC and TA subset of cells (according to SingleR cell type annotation) on a pseudotime trajectory **(H)** Differential cell type abundance analysis of DAT treated organoids compared to their respective control or between treated WT and GT organoids, based on negative binomial regression. Data shown as log₂ fold change regression estimate with associated 95% confidence interval based on the normal distribution. P-values were adjusted for multiple testing using the FDR approach, controlling for an FDR of 10%. Data are shown as mean ± SEM if not stated otherwise.

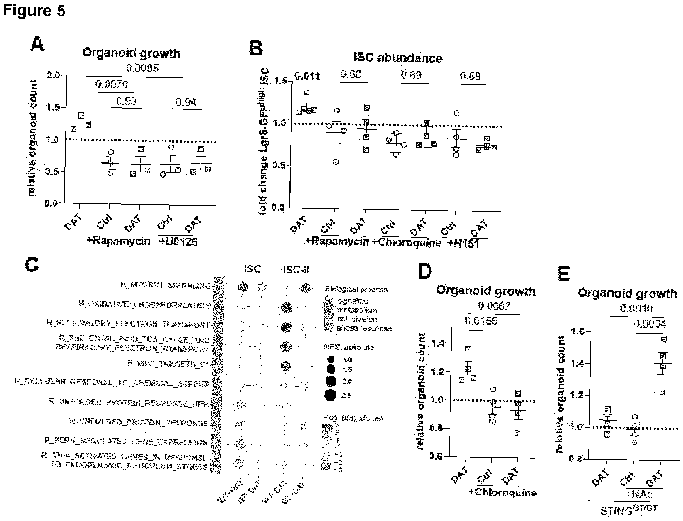

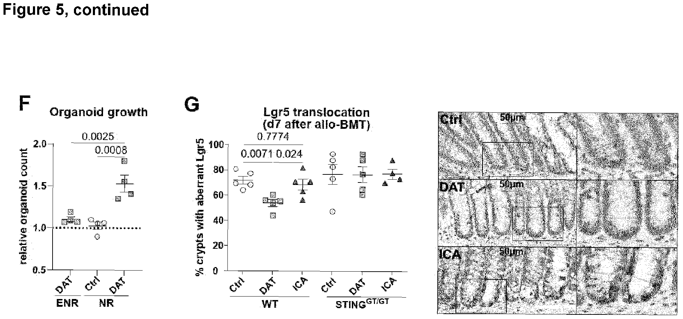

**Fig. 5****: DAT activated mTORC1 signaling in intestinal stem cells is counter-regulated by STING. (A)** Murine SI organoid growth was assessed as in Fig. 2D but in presence of the mTOR inhibitor Rapamycin (1 µM) or the MAPK inhibitor U0126 (10 µM). Pooled data from 3-4 independent experiments was analysed by ordinary one-way ANOVA with Dunnett's correction for multiple comparisons or unpaired t-test. **(B)** Lgr₅-GFP^{high} ISC abundace were assessed as in Fig. 2C, but in presence of rapamycin, the STING inhibitor H151 or chloroquine. Pooled data from 4 independent experiments. P-values were calculated using one sample t-test or Mann-Whitney U test. **(C)** Dotmap of GSEA results of selected pathways/gene sets for different cell types and treatments (vs. control, cell types as described in Fig. 4F+G). Dots are colored by the GSEA negative log₁₀ of the GSEA q-value (FDR), the sign indicates the direction of the regulation (up positive, down negative). The size of the dots corresponds to the NES. Gene sets/pathways are derived from the Hallmark (H) and Reactome (R) gene set collections of MSigDB. **(D)** Murine SI organoid growth was assessed as in Fig. 2D but in presence of the autophagy inhibitor chloroquine (10µM). **(E)** STING deficient SI organoids were stimulated as in Fig. 4A but in presence of the antioxidant agent N-acetyl-cystein (2.5 mM). Pooled data from 4 independent experiments. **(F)** Murine SI organoid growth was assessed as in Fig. 2D in ENR media (containing EGF, Noggin and R-spondin) or NR (without EGF). Pooled data from 4 independent experiments. **(G)** C57BL/6 WT and STING Goldenticket mice underwent metabolite treatment and allo-BMT as in Fig. 4D. The localization of *Lgr₅* expression was assessed in large intestinal crypts by in-situ hybridization. Percentage of crypts with *Lgr₅* expression in the upper crypt (above +4 position) and representative images are shown. Data from 2 independent experiments (n=5). P-values were generated by ordinary one-way ANOVA with Dunnett's correction for multiple comparisons (A, D, E, F, G) or unpaired t-test (A). All data are shown as mean ± SEM.

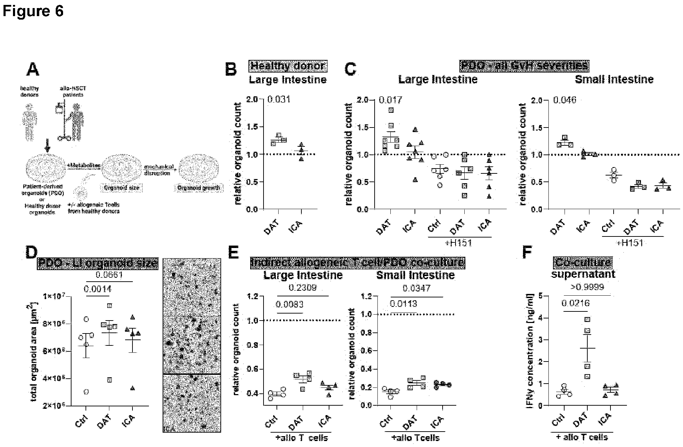

**Fig. 6****: DAT treatment is effective in healthy donor and patient-derived human organoids.**
   **(A)** Experimental overview. **(B)** Human LI organoids derived from healthy donors were stimulated with metabolites and their growth assessed. Pooled data from 3 different donors in independent experiments. **(C)** Human LI and SI organoids derived from patients receiving allo HSCT were stimulated with metabolites as in (A) or in presence of H151. Pooled data from 3-7 donors in independent experiments. **(D)** Size of LI PDO after 6 days of metabolite treatment assessed as total organoid area. Pooled data from 5 independent experiments. **(E)** Human organoid/T cell co-culture without direct interaction. To mimic cytokine-mediated toxicity, LI PDO were co-cultured with CD3/28 beads + 30 U/ml IL-2 activated panT cells derived from healthy volunteers in the surrounding media for 4 days and their growth assessed as in (A). Counts are shown relative to untreated organoids. Pooled data from 4 different patients in independent experiments. **(F)** IFNγ concentration in cell culture supernatants of organoid/T cell co-cultures assessed by cytokine bead array. N=4 wells from two donors. P-values were calculated by one-sample t-test (B+C), ordinary one-way ANOVA with Dunnett's correction for multiple comparisons (E+F) or paired one-way ANOVA with Dunnett's correction for multiple comparisons (D).

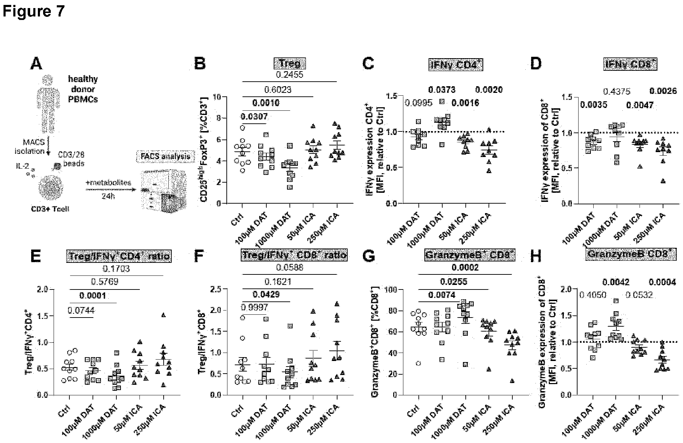

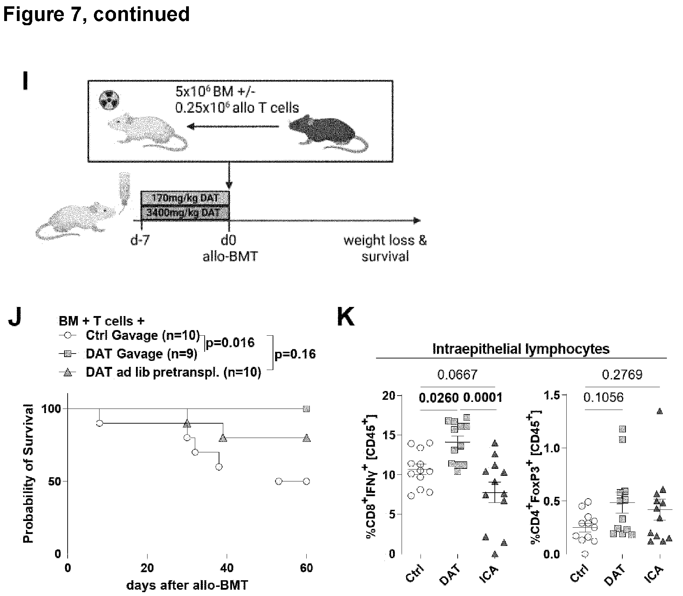

**Fig. 7****: DAT promotes T cell activation in vitro in humans and in vivo in mice. (A)** Experimental overview. Human pan-T cells were stimulated with human anti-CD3/28 beads, 30 U/ml hIL-2 and indicated concentrations of metabolite for 24 hours and analyzed by flow cytometry. Abundance of the indicated T cell subpopulations **(B+G),** protein expression according to mean fluorescence intensity (MFI) normalized to the respective control **(C-D+H)** or ratio of Tregs to IFNy+CD4+ or IFNy+CD8+ T cells **(E-F).** Pooled data from 10 healthy individuals. Data was analyzed using ordinary one-way ANOVA for paired data with Dunnett's correction for multiple comparisons. **(I)** Experimental in *vivo* design. **(J)** Survival of mice. Ctrl and DAT gavage mice are also shown in Fig. 1E. Data was analyzed by Log-Rank test from 2 independent experiments. **(K)** BALB/c mice were orally treated with the metabolites and underwent allo-BMT as depicted in Fig. 1D. On day 7 mice were sacrificed and H-2K^{b+} donor T cells were analyzed by flow cytometry. Abundance of IFNy+CD8+ cytotoxic T cells and abundance of regulatory T cells. Pooled data from 3 independent experiments and n=12 mice. Data was analyzed using ordinary one-way ANOVA with Dunnett's correction for multiple comparisons. If not depicted otherwise, all data are shown as mean ± SEM.

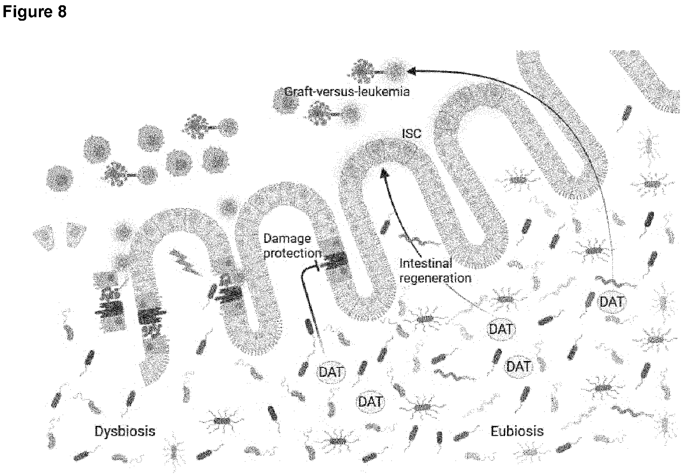

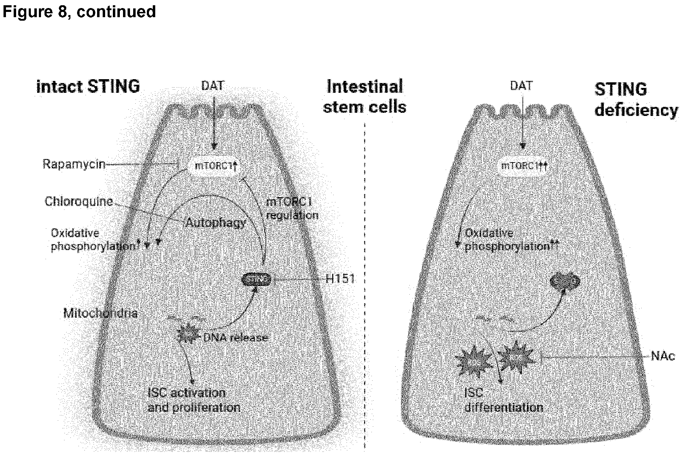

**Fig. 8****: Graphical summary of effects and mechanisms described herein.**

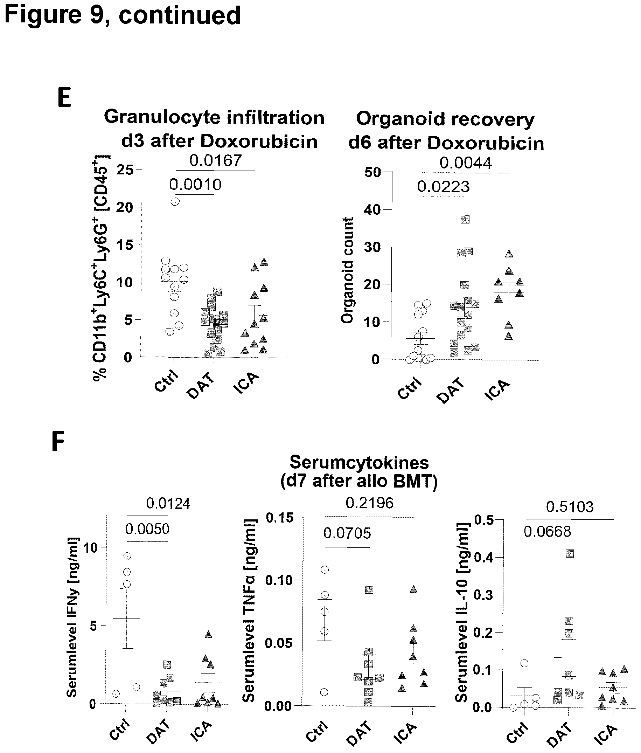

**Fig. 9****: *In vitro* organoid and in *vivo* data. (A)** Surface expression of MHC class II on EpCAM+ epithelial cells after IFNγ stimulation. Murine small intestinal organoids were seeded and stimulated with indicated metabolites or control for 3 days. Afterwards, indicated groups were stimulated with 2.5ng/ml IFNγ alone or in combination with metabolites. The expression of MHC II was determined after additional 3 days of IFNγ stimulation. **(B)** Left: Number of viable organoids alone or after irradiation with 4 Gy with additional metabolite or control treatment assessed on day 6 of culture. N=8 wells from 2 independent experiments. P-values were calculated with ordinary one-way ANOVA with Dunnett's correction for multiple comparisons. Right: Organoid numbers were assessed after passage. Pooled data from 4-6 experiments. P-values were calculated by ordinary one-way ANOVA with Dunnett's correction for multiple comparisons. **(C)** Count of regenerated large intestinal organoids from 300 seeded crypts per drop. Pooled data from 3 independent experiments (n=9-10). P-values were calculated by ordinary one-way ANOVA with Dunnett's correction for multiple comparisons. **(D)** Number of viable organoids generated from 200 crypts isolated on day 7 after TBI. Pooled data from 4 (n=14-19) independent experiments. P-values were calculated by ordinary one-way ANOVA with Dunnett's correction for multiple comparisons. **(E)** Granulocyte infiltration (left) and organoid recovery (right) after intestinal mucositis was induced by intraperitoneal injection of the chemotherapeutic doxorubicin. C57BL/6 mice were treated with metabolites from day 7 before damage induction and intraperitoneal injections of doxorubicin were performed on day 0. Barrier damage was assessed on day 3 by granulocyte infiltration and organoid recovery was assessed on day 6 after doxorubicin injection. P-values were calculated by ordinary one-way ANOVA with Dunnett's correction for multiple comparisons. **(F)** Serum cytokine level determined in C57BL/6 mice treated as in Fig. 2 G by cytokine bead array. P-values were calculated by ordinary one-way ANOVA with Dunnett's correction for multiple comparisons.

In the following, reference is made to the examples, which are given to illustrate, not to limit the present invention.

### EXAMPLES

### Example 1: Methods

### Patient cohort

The research in humans reported here complies with all relevant ethical regulations. Study protocols were approved by the institutional review boards (IRB) "Ethikkommission der TU München" (295/18 S) and "Ethikkommission der Universität Regensburg" (14-47_1-101, 14-101-0047, 21-2521-01). The study is registered at the German Clinical Trials Register (DRKS00034175).

All patients receiving allo-SCT at the University Hospital of the Technical University Munich (between 2019 and 2021) and University Hospital Regensburg (between 2018 and 2021) were screened. Patients were enrolled after obtaining informed consent and in accordance with the IRB-approved study protocols referenced above. Patients did not receive financial participant compensation.

The study cohort included female and male patients. Sex (biological attribute) was assigned based on electronic patient records. The inventors did not consider sex in the study design and did not perform separate analysis according to sex, since sex did not affect microbiome-predicted outcomes in other cohorts of allogeneic stem-cell transplantation patients. At the University Hospital Regensburg, ethnicity was determined by self-reporting, all patients identified as "Non-Hispanic/white".

Fecal samples were collected once a week, beginning on day -7 before allo-SCT and ending on day +21 after allo-SCT. Samples were obtained on one to two days around the specified time points. Stool samples were collected as unprocessed, native samples and transferred to the laboratory within four hours, where they were stored at -80°C until further processing.

Patients with a minimum of three longitudinal samples were included in the analysis. No statistical method was used to predetermine sample sizes, but the size of the inventors' cohort is similar to those reported in previous publications. Randomization did not apply in the inventors' non-interventional cohort. Data collection was not performed blind, and investigators were not blinded to outcome assessment.

**Table 1: Patient characteristics: HSCT denotes hematopoietic stem cell transplantation, SE standard error, MDS myelodysplastic syndrome, MPN myeloproliferative neoplasia, NHL Non-Hodgkin lymphoma, PBSC peripheral-blood stem cell, BM bone marrow, and TRM transplantation-related mortality. Diseases categorized as "other" include aplastic anemia and myelosarcoma.**

| **Patients - no.** | | **50** |
|---|---|---|
| **Mean age at HSCT - yrs ± SE** | | 56.5 ± 1.5 |
| **Male/Female Sex - no. (%)** | | 27 (54)/23 (46) |
| **Diagnosis - no. (%)** | Acute leukaemia | 22 (44) |
| | MDS/MPN | 19 (38) |
| | NHL | 5 (10) |
| | other | 4 (8) |
| **Donor type - no. (%)** | Unrelated | 35 (70) |
| | Sibling | 5 (10) |
| | Haploidentical | 10 (20) |
| **Conditioning - no. (%)** | Ablative | 7 (14) |
| | Reduced intensity | 43 (86) |
| **Stem cell source - no. (%)** | PBSC | 42 (84) |
| | BM | 8 (16) |
| **High DAT levels - no. (%)** | | 22 (44) |
| **High ICA levels - no. (%)** | | 10 (20) |
| **2 year relapse incidence (%)** | | 10 (20) |
| **2 year TRM incidence (%)** | | 10 (20) |
| **2 year survival (%)** | | 31 (62) |

### Quantification of fecal metabolites in human samples

Metabolite levels at days -7 and +7 to +21 relative to allo-HSCT were used for the analysis. When more than one sample per patient was available, the mean metabolite concentration was used. Patients were stratified into high and low DAT and ICA groups, respectively.

### Analysis of Clinical Outcomes

Overall survival at a follow-up of two-years was analyzed using the Kaplan-Meier estimator. Cumulative incidence of relapse was estimated accounting for its competing risk transplantation-related mortality (TRM). Cumulative incidence functions between groups were tested for equality using Gray's test and the hazard ratio for the different endpoints was calculated in R based on the Fine and Gray model using the crr function and the Cox regression model (OS) using SPSS v29.

### Mice

C57BL/6J and BALB/c mice were purchased from Janvier-Labs. Mice genetically modified for the interferon-a receptor 1 (B6(Cg)-Ifnar1tm1.2Ees/J; RRID: IMSR_JAX:028288; here referred to as IfnaR^{-/-}), STING (C57BL/6J-Sting1gt/J; RRID:IMSR_JAX:017537; here referred to as STING Goldenticket, STING^{GT}/^{GT}), MAVS (B6;129-Mavstm1Zjc/J; RRID:IMSR_JAX:008634; here referred to as MAVS^{-/-}) and Lgr₅-GFP (B6.129P2-Lgr5tm1(cre/ERT2)Cle/J; RRID:IMSR_JAX:008875) have been described previously and were breed in the animal facilities of the University Hospital Regensburg or Technical University Munich. Germfree BALB/c mice were bred and maintained in special isolators at the GF mouse facility of the University of Regensburg and transferred to individual sterile isocages at the beginning of experiments. Transplantation and treatment with metabolites was performed under sterile conditions in a laminar flow. GF status was confirmed at the beginning and the end of each experiment.

Animals were housed in specific pathogen-free (SPF) animal research facilities and monitored for pathogens according to FELASA recommendations. Mice were between 6 and 10 weeks old at the beginning of the experiments. Mice were maintained on a 12-h light-dark cycle, a constant temperature of 24 °C and a humidity level of 55%-65%. *Ad libitum* access to autoclaved standard laboratory chow and water was guaranteed at any time. Environmental enrichment, including nesting material, houses, and chew toys, was provided to promote their welfare and well-being. The animals were housed in the facility where the experiments took place for at least one week prior to the start of an experiment for acclimatisation. Genetically modified mice were co-housed with age- and sex-matched WT mice for a period of at least 4 weeks before starting in *vivo* experiments. Treatment groups were not co-housed to avoid microbial/metabolite transfer through coprophagy between groups. Animal studies were approved by the local regulatory authorities (Regierung von Oberbayern, ROB-55.2-2532.Vet_02-15-09, ROB-55.2-2532.Vet_02-20-168 or Regierung von Unterfranken, RUF-55.2.2-2532-2-1301).

### Media and reagents

RPMI-1640 (Invitrogen) was supplemented with 10% (v/v) FCS (Gibco), 2 mM L-glutamine, 100 U/ml penicillin and 100 µg/ml streptomycin (Sigma-Aldrich). Complete RPMI contained 10% (v/v) FCS, 2 mM l-glutamine, 100 U/ml penicillin, 100 µg/ml streptomycin, 50 µM β-mercaptoethanol (Gibco), 1x non-essential amino acids (Gibco), 1 mM sodium pyruvate (Gibco) and 10 mM HEPES (Gibco). Human T cell media contained 10% (v/v) FCS, 2 mM 1-glutamine, 100 U/ml penicillin, 100 µg/ml streptomycin, 1 mM sodium pyruvate (Gibco), 10 mM HEPES (Gibco) and was supplemented with 30U/ml hIL-2 (Peprotech).

ENR media contained advanced DMEM/F12 (Life technologies), 2 mM L-glutamine (Sigma), 10 mM HEPES (Life technologies), 100 U/ml penicillin, 100 µg/ml streptomycin (Life technologies), 1.25 mM N-acetyl cysteine (Sigma), 1x B27 supplement (Life technologies), 1x N2 supplement (Life technologies), 50 ng/ml mEGF (Peprotech), 100 ng/ml rec. mNoggin (Peprotech), 10% human R-spondin-1-conditioned medium of hR spondin-1 transfected HEK 293T cells. In indicated experiments EGF-depleted ENR (indicated as NR media) was used that contained all mentioned ingredients besides EGF. Additional blocking of organoid-produced EGF was achieved by supplying 0.5 µg/ml aEGF antibody (Bio-Techne). 2.5mM N-acetyl-cystein was used in NAc high ENR media. WENR contained advanced DMEM/F12 (Life technologies), 2 mM L-glutamine (Sigma), 10 mM HEPES (Life technologies), 100 U/ml penicillin, 100 µg/ml streptomycin (Life technologies), 1.25 mM N-acetyl cysteine (Sigma), 1x B27 supplement (Life technologies), 50 ng/ml hEGF (Peprotech), 100 µg/ml Normocin (InvivoGen), 50% L-WNR-conditioned medium of L-WRN cells. Human or murine IntestiCult intestinal organoid growth media (STEMCELL Technologies) was supplemented with 100 U/ml penicillin and 100 µg/ml streptomycin.

Gavage solution of DAT was prepared with 17 mg/ml 3-(4-hydroxyphenyl)-propionic acid (DAT, 98%, Sigma-Aldrich) in 5% EtOH (Carl Roth) in water. Drinking water was prepared with 100 mM DAT and neutralized with NaOH (Merck) in tap water. The respective solvents served as controls. ICA was prepared with 15 mg/ml. Gavage solutions of both metabolites were thereby prepared in equimolar concentrations. Daily in *vivo* uptake of metabolites was estimated at 170 mg/kg body weight (DAT gavage), 3400 mg/kg body weight (DAT drinking water with 4 ml water uptake/20 g mouse) or 150 mg/kg body weight (ICA). Gavage solutions and drinking water were filtered through 0.22 µm sterile filters and were prepared freshly every 3 days. Broad-spectrum antibiotics with 0.5 g/L Ampicillin and 0.25 g/L enrofloxacin or 0.625 g/L Meropenem were administered via the drinking water (ad libitum). Drinking water supplemented with antibiotics was replaced every 2 days to maintain constant antibiotic activity.

### GvHD induction after allo-BMT

For GvHD induction after allo-BMT a major mismatch (H-2k^{d}/H-2k^{b}) with myeloablative TBI was used. BALB/c recipients were irradiated with 8 Gy and after 3-4 hours intravenously injected with 5x10⁶ allogeneic (C57BL/6J) T-cell-depleted bone marrow cells alone or in combination with 0.25x10⁶ allogeneic T cells. Bone marrow cells were isolated from naive C57BL/6J mice and TCD was performed using CD90.2 MicroBeads (Miltenyi Biotec). Donor T cells were isolated from pooled spleens of naive C57BL/6J mice using CD4 and CD8 MicroBeads (Miltenyi Biotec) respectively and mixed in equal proportions. Recipient mice were monitored daily in a non-blinded manner and euthanized when they reached one of the human end point criteria: 1) more than 30% weight loss; 2) bloody diarrhea; 3) apathy or if other determination criteria were met according to standard legal procedure.

### Graft-versus-leukemia model

Ba/F3 cells (murine, DSMZ no.: ACC 300) transfected with the FLT3-ITD oncogene as well as the luciferase (luc)⁺ and green fluorescent protein (GFP)⁺ serving as marker genes were used. BALB/c mice received 8 Gy TBI and after 3-4 hours were intravenously injected with 5x10⁶ allogeneic (C57BL/6J) BM cells and 500-750 tumor cells. On day 2 post transplantation, 0.25x10⁶ allogeneic T cells were transplanted. BM only mice received no additional T cells. Expansion of leukemia cells was monitored by bioluminescence imaging.

### Bioluminescence imaging (BLI)

For in *vivo* BLI, mice were injected intraperitoneally (i.p.) with luciferin (150 mg/kg body weight) and narcotized by inhalation with 2.5% isoflurane. Imaging was performed with the IVIS 50 bioluminescence imaging system (Xenogen) and quantified using the Living image Software (Xenogen).

### Induction of intestinal damage by total body irradiation, doxorubicin or allo-BMT

Following in *vivo* models for intestinal damage were used:
To induce damage by total body irradiation, C57BL/6 mice were irradiated with 11 Gy and subsequently intravenously injected with 5x10⁶ syngeneic (C57BL/6) BM cells.

Intestinal damage by Doxorubicin was induced in C57BL/6 mice by intraperitoneal injection of 7.5 mg/kg (d3 granulocyte infiltration assay) or 20 mg/kg (d6 organoid recovery assay) doxorubicin.

Generally, allogeneic tissue damage was induced in C57BL/6 mice following 11 Gy TBI by intravenous injection with 5x10⁶ allogeneic (BALB/c) BM cells alone or in combination with 2x10⁶ allogeneic T cells. Due to increased susceptibility to intestinal tissue damage, the T cell dose was reduced to 1.5x10⁶ allogeneic T cells in IFNaR^{-/-} and meropenem-treated mice. Germfree BALB/c mice were transplanted with 0.4x10⁶ allogeneic (C57BL/6) T cells and 5x10⁶ BM cells following 8 Gy TBI.

### Fecal microbiota transfer

C57l/6 mice received the antibiotics ampicillin and enrofloxacin for 5 days via drinking water. On days 2 and 5 after completion of antibiotics, mice received suspensions of the indicated bacterial strain (DSM 6740, DSM 26117, German Collection of Microorganisms and Cell Cultures GmbH) or 20 mg of cecal content from SPF mice donors as control via oral gavage and rectal application. Mice received 7.5x10⁸ bacteria in a 150µl volume by 2/3 oral gavage and 1/3 rectal application. Bacterial strains were expanded under strictly anaerobic conditions in Anaerobe Basal Broth (ABB; Thermo Scientific/Oxoid # CM0957). The concentration of each bacterial species was quantified based on OD600. Allo-BMT was performed on day 7 post-antibiotic treatment. Colonization was confirmed by performing qPCR using F. plautii-specific primers from stool pellets.

### qPCR for fecal bacterial quantification

Nucleic acids were extracted from fecal stool pellets using the MagNA Pure 24 instrument and the MagNA Pure 24 Total NA Isolation Kit (Roche Diagnostics, Rotkreuz, CH) following repeated bead beating with Lysing Matrix Y beads (MP Biomedicals). Flavonifractor plautii detection in fecal DNA extracts was performed with FP_F and PF_R primers. Cloned PCR fragments amplified from F. plautii ATCC 6740 genomic DNA served as quantification standards. Quantitative PCR was conducted on a LightCycler 480 II instrument (Roche) using the LightCycler 480 SYBR Green I Master Kit (Roche) under the following conditions: 40 cycles (95 °C for 10s, 60 °C for 15s, 72 °C for 15s) with an initial 10-minute hot start at 95 °C. Total 16S rDNA copies were determined.

### Intestinal immune cell preparation

For intestinal immune cell preparations, ileum (defined as distal 10 cm of small intestine) was flushed with cold PBS, longitudinally opened to remove remaining content and cut into 1 cm pieces. After washing, the lamina propria dissociation kit (Miltenyi Biotec) was used according to manufactures instructions. Leucocytes were enriched on a 20/40/80% Percoll gradient (GE Healthcare Life Science, Pittsburgh, PA).

### Granulocyte infiltration assay

Ileal leucocytes were isolated as described above. Lamina propria cells were collected, washed with PBS and subsequently analyzed by flow cytometry.

### Crypt isolation

Large or small intestine were flushed with PBS, longitudinally opened to remove remaining content and cut into 1 cm pieces. After washing until the supernatant remained clear, tissue pieces were incubated in PBS containing ethylenediamine-tetraacetic acid (EDTA) at 4°C. Small intestine was incubated for 30 minutes in 5 mM EDTA, large intestine was incubated 3 times for 10 minutes with 30 mM EDTA. Small intestinal crypts were dissociated by forceful shaking 10-times. Large intestinal crypts were dissociated by 5-times forceful shaking for 30 seconds. Crypt-containing supernatant was filtered through one 100 µm and two 70 µm strainers or until the supernatant was clear.

For crypt isolation from human intestinal biopsies, the tissue pieces were washed twice in PBS and then digested with collagenase (Sigma Aldrich, C969₇-₅0MG, 2 mg/ml in RPMI) for 30 minutes at 37°C. Afterwards, the digestion solution was carefully removed, PBS added and the tissue disrupted by forceful pipetting. The crypt suspension was passed through a 100 µm strainer, resuspended in 50-100 µl of Matrigel and seeded into organoid cultures. The culture was started in human IntestiCult intestinal organoid growth media additionally supplemented with normocin (Invivogen, ant-nr-1, 100 µg/ml) and gentamicin (Gibco, 20µg/ml) and then kept in WENR media.

### Organoid recovery assay

Intestinal crypt isolation was performed as described above. Time points of crypt isolation and used organs are depicted in the figure legends. After isolation, crypts were counted and a defined number (n=200 crypts/drop for small intestine, n=300 crypts/drop for large intestine) of crypts per drop used to initiate organoid cultures. At least two drops per mouse were seeded. Small intestinal organoids were cultured in ENR media. When small intestinal crypts were isolated from germfree or broadband antibiotic-treated mice, ENR media was supplemented with 10 µM Rock-inhibitor Y27632 to ensure stable organoid formation. Large intestinal organoids were cultured in IntestiCult intestinal organoid growth media supplemented with Rock-inhibitor Y27632. Established organoids were counted 3 days after seeding.

### Immunohistochemistry

After deparaffinization and re-hydration, slides were subjected to heat-induced antigen retrieval in a citrate-based buffer, pH 7.2 for 30 minutes using a microwave at 320 W. Blocking was done with peroxidase blocking solution, Dako REAL (cat no. S2023). Immunohistochemistry (IHC) was performed using the following primary antibodies: CD₃ (1:50; clone SP7, catalog no. RM-9107-S1, Thermo Fisher). Primary antibodies were diluted in Antibody Diluent, Dako REAL (cat no. S2022) and incubated for 45 to 60 minutes at room temperature. Slides were washed using wash buffer from Dako/Agilent (10x concentrate, cat no. S3006). Secondary antibody (ready to use, cat no. 414341F, Histofine) was incubated for 45 to 60 minutes at room temperature. Detection was done using the DAB+ substrate detection system (catalog no. K3468, Dako). All stains were validated by internal and/or external positive controls as well as negative control specimens. The slide scanner Pannoramic 1000 Flash RX^{®} (Sysmex Europe SE) was used for image acquisition choosing the 20x objective. Virtual images from the slide scans were extracted for demonstration purposes using the software CaseViewer version 2.4 (Sysmex Europe SE).

The degree of CD₃+ T-cell infiltration was examined in a blinded fashion by a board-certified pathologist. The degree of epithelial T cell infiltration was assessed by counting the number of infiltrating CD3+ cells (intraepithelial lymphocytes) in 10 high power fields (HPF) and calculating the average number of infiltrating cells per HPF for each specimen.

The GvHD pathology was examined in a blinded fashion by a board-certified pathologist (A.M. and D.H.). Histopathologic scores were assigned based on established criteria.

### Lgr₅ in situ hybridization

For detection of Lgr₅ expression in FFPE mouse tissue, in situ hybridization (ISH) was performed using RNAscope^{®} 2.5 HD Reagent Kit-BROWN (322300; ACD, Hayward, CA, USA) with the RNAscope^{®} Probe- Mm-Lgr₅ (312171; ACD) specific for Lgr₅ RNA in mouse. Tissue sections of 4 µm thickness were deparaffinized in xylene and ethanol and blocked with peroxidase (10 min). Slides were boiled in kit-provided antigen retrieval buffer at 95°C for 15 minutes and digested afterwards with protease at 40°C for 30 minutes. For hybridization tissue sections were incubated with the target probe in the HybEZ hybridization oven (ACD) at 40°C for 2 hours. Pre-amplification and amplification steps were conducted using kit-provided reagents according to the manufacturer's recommendations. For signal detection sections were incubated with the BROWN Reagent for 10 minutes at room temperature followed by counterstaining with hematoxylin, dehydration in ethanol and xylene and mounting with a xylene-based mounting medium.

### Mucus staining

Colonic sections containing stool pellets were collected 2 cm from the anus and transferred into fresh methanol-Carnoy's fixative (60% methanol, 30% chloroform, 10% acetic acid) for 24h at room temperature. Tissue was then incubated twice with methanol for 30 minutes, twice in ethanol for 20 minutes, twice in xylene for 25 minutes and 1 hour in paraffin. 5µm-thick sections were used for mucus staining by Periodic acid-Schiff staining. To assess the thickness of the mucus layer, 8 evenly distributed sections were evaluated.

### In vitro T cell activation

For *in vitro* T cell assays, human pan T cells were isolated from PBMCs of healthy volunteers with the human Pan T Cell Isolation Kit (Miltenyi Biotec) according to manufacturer's instructions. For stimulation, 2x10⁵ T cells were seeded in a 96-well cell culture treated U-bottom well in T cell media and supplemented with 30 U/ml hIL-2 (Peprotec) and 2 µl of Dynabeads^{™} Human T-Activator CD3/CD28 beads (Thermo Fisher). Metabolites were added as indicated in the figure legends. After 24 h incubation at 37 °C and 5% CO2, cells were harvested and stained for subsequent flow cytometry analysis.

### In vitro APC isolation

For in vitro activation of human APCs, CD14+ monocytes were isolated from PBMCs of healthy volunteers with the human CD14 microbeads (Miltenyi Biotec) according to manufacturer's instructions. CD14+ monocytes were either stimulated directly or differentiated into moDCs using the human Mo-DC Differentiation Medium (Miltenyi Biotec) according to manufacturer's instructions.

### In vitro organoid culture

Crypts were isolated as described above and suspended in liquefied growth factor reduced Matrigel (Corning) (33% ENR-medium or PBS; 66% growth factor-reduced Matrigel) at 4°C, and were plated in delta-surface Nunc 24-well plates in 30 µl drops, each containing approximately 200 crypts. All plates were incubated at 37 °C / 5% CO2 and ENR medium was replaced every 2-3 days. After 7 days organoids were passaged by mechanical disruption with a 5 ml syringe and an 18 G needle.

For stimulation experiments, established organoids beginning after the first passage were used. In some experiments, the inventors directly stimulated intestinal crypts after isolation without prior establishing time (referred as *ex vivo* crypt stimulation). To initiate stimulation experiments, organoids were passaged and seeded. Microbial metabolites were added after seeding (100 µM DAT or 50 µM ICA). In experiments using inhibitors, rapamycin (Invivogen, 1onM-1uM), U0126 (Invivogen, 10µM), chloroquine (1µM) or H-151 (Invitrogen, 1µg/ml) were added 4 hours after seeding to prevent direct effects on the organoid formation after passaging. Metabolites were added 2 hours after the inhibitors. For the evaluation of the MHC II expression on organoids, organoids were stimulated with metabolites for 3 days. Afterwards, organoids received additional stimulation with 2.5 ng/ml mIFNγ (Peprotec). Pictures for size determination were taken on day 6 of stimulation and analyzed in a blinded fashion using the ImageJ software. Flow cytometry analysis was performed on day 6 stimulation. On day 7, stimulated organoids were passaged. After 3 additional days, the number of established organoids after passage was assessed and normalized to the control group.

Human organoid cultures were performed with large and small intestinal organoids derived from tissue biopsies. Organoids were used from passage 3 onwards and cultured in WENR media that was replaced every other day. Otherwise identical procedures as for murine organoids were used.

### T cell/organoid co-cultures without direct interaction

For murine co-cultures, T cells were isolated from splenocytes of donor BALB/c mice using the Pan T Cell Isolation Kit II, mouse (130-095-130, Miltenyi Biotec) according to the manufacturer's protocol. For co-culture, Jx10⁴ allogeneic T cells were added to passaged C57BL/6 small intestinal organoids with murine IL-2 (30 U/ml, Peprotec) and Dynabeads murine T-activator CD3/CD28 (ThermoFisher, 2 µl per well). To prevent direct contact between organoids and T cells, plates were kept slightly tilted with the organoid drop above the T cells placed at the bottom of the well. After 4 days, T cells were removed and media exchanged to ENR. Photos for size determination were taken at day 6 and area size of organoids was analyzed in a blinded fashion using the ImageJ software. Organoids were passaged after 7 days and counted on day 3 after passage. Before passaging, all organoid drops were microscopically inspected to exclude T cell infiltration into to the organoid drops.

For human co-cultures, a similar procedure was used. PBMCs from healthy human donors were isolated with Biocoll cell separation solution and T cells isolated with the human Pan T Cell Isolation Kit (Miltenyi Biotec) according to manufacturer's instructions. Jx10⁴ allogeneic T cells were co-cultured with passaged organoids, human IL-2 (30 U/ml, Peprotec) and Dynabeads human T-activator CD3/CD28 (ThermoFisher, 2 µl per well).

### In vitro intestinal GVHD model

Small intestinal crypts of BALB/c mice were isolated and organoid cultures initiated as described above. For isolation of small intestinal intraepithelial lymphocytes (IEL), small intestine of C57BL/6 mice was removed, washed with PBS, cut into 0.5 cm pieces- and twice incubated in HBSS (Sigma-Aldrich) + 10 mM HEPES + 5 mM EDTA + 5% FCS + 1mM dithiothreitol (Sigma-Aldrich) for 20 minutes at 37°C and for additional 20 minutes at 37°C in HBSS + 10 mM HEPES. After each incubation step, the tissue pieces were vortexed and the supernatant containing IEL was collected. T cells were then enriched by a 40/70% Percoll gradient followed by a positive magnetic cell separation using anti-CD3-PE antibodies (Biolegend) and anti-PE MicroBeads, ultrapure (Miltenyi Biotec).

To set up the allogeneic co-culture, organoids were removed from Matrigel 2-4 days after crypt isolation. Approximately 100 organoids were incubated with 2.5x10⁵ CD3+ magnetically enriched IEL T cells for 30 minutes at 37°C. Afterwards, organoids and T cells were embedded in Matrigel and cultured in organoid media additionally containing 10 ng/ml mIL-7 (Immunotools), 10 ng/ml mIL-15 (Immunotools) and 100 U/ml hIL-2 (Immunotools). Cell death in organoids was assessed after 2 days.

To assess cell death, organoids were stained with 10 µg/ml propidium iodide (Invitrogen) and 10 µg/ml Hoechst 33342 (Invitrogen) for 30 minutes at 37°C. Propidium iodide and Hoechst 33342 fluorescence were determined with the Tecan infinite M200 plate reader. To quantify cell death independently of the seeding density, intensity signal of the cell-impermeable dye propidium iodide as a surrogate for the amount of cell death was normalized to the fluorescence intensity of the cell-permeable dye Hoechst representing the number of cells in each well. Subsequently, to correct for physiological baseline cell death, the measured basal cell death, i.e. PI/Hoechst ratio was further normalized to the organoid control without T cells (i.e. normalized to BALB/c organoids cultured alone). Representative images of stained organoids were taken on a Leica DMI4000 B inverted microscope with a 10X objective.

### Organoid irradiation

Small intestinal organoids were passaged and seeded as described above and stimulated as indicated. After 4h, organoids were irradiated with 4 Gy. The number of viable organoids was assessed on d6 after irradiation. The number of regenerated organoids after passage was assessed 3 days after passage on day 7 after irradiation.

### Flow cytometry

For intracellular cytokine staining, cells were stimulated for 3-4h with eBioscience^{™} Cell Stimulation Cocktail (plus protein transport inhibitors) (#00-4975-93). Cell suspensions were stained in PBS. Fluorochrome-coupled antibodies were purchased from eBioscience, BD or BioLegend. Data were acquired on a FACS LSR Fortessa X-20 or Symphony A₅ (BD Biosciences) and analyzed using the FlowJo software (TreeStar). All staining protocols included 20 min staining with a live/dead marker and CD16/CD32 antibody (Biolegend) at 4 °C and surface marker staining for 30 min at 4°C. For intracellular cytokine staining, the Foxp3 Transcription Factor Fixation/Permeabilization Kit (eBioscience) was used for fixation and permeabilisation. Intracellular staining was performed for 60 minutes at 4 °C.

### Cytokine measurements

Cytokine levels in cell culture supernatants or murine serum samples were determined using the LEGENDplex^{™} Mouse Inflammation Panel (13-plex) or the LEGENDplex^{™} Human AntiVirus Response Panel (both Biolegend) according to manufacturer's instructions.

### Cell sorting

Single cell suspensions of small intestinal organoids were generated by removing organoids from the culture with PBS and digestion with TrypleE Express for 10 minutes at 37°C. Single cells were passed through a 100µM strainer, washed and stained with EpCAM-BUV395 (BD Bioscience) and respective hashing antibody (TotalSeq-Bo301-306 anti-mouse Hashtag 1-6 Antibody, Biolegend). Live/dead staining was performed immediately before sorting by adding propidium iodide. Single cells were sorted as live/dead-EpCAM+ cells.

### Metabolome Analysis

### Sample preparation for targeted analysis

Approximately 100 mg of human stool was weighed in a 15 ml bead beater tube (TeenPrepTMLysing Matrix D, MP Biomedical) and extracted with 5 ml of methanol. The samples were extracted with a bead beater (FastPrep-24^{™} 5G, MP Biomedicals) supplied with a CoolPrepTM cooling module (MP Biomedicals, cooled with dry ice) 3 times each for 20 seconds with 30 seconds breaks in between a speed of 6 m/sec. 1 ml of the fecal suspension was dried in a vacuum centrifuge (Eppendorf Vacufuge) to determine the dry weight.

### Measurement of SCFA, lactic acid, desaminotyrosine and indole-3-carboxyaldehyde

The 3-NPH method was used for the quantitation of desaminotyrosine and indole-3-carboxyaldehyde. Briefly, 40 µl of the fecal extract and 15 µl of 50 µM isotopically labeled standards were mixed with 20 µl 120 mM EDC HCl-6% pyridine-solution and 20 µl of 200 mM 3-NPH HCL solution. After 30 min at 40°C and shaking at 1000 rpm using an Eppendorf Thermomix (Eppendorf, Hamburg, Germany), 900 µl acetonitrile/water (50/50, v/v) was added. After centrifugation at 13000 U/min for 2 min the clear supernatant was used for analysis. The measurement was performed using a QTRAP 5500 triple quadrupole mass spectrometer (Sciex, Darmstadt, Germany) coupled to an ExionLC AD (Sciex, Darmstadt, Germany) ultrahigh performance liquid chromatography system. The electrospray voltage was -4500 V, curtain gas 35 psi, ion source gas 1 55 psi, ion source gas 2 65 psi and the temperature 500°C. The MRM-parameters were optimized using commercially available standards. The chromatographic separation was performed on a 1.7 µm, 100 × 2.1 mm, 100 Å Kinetex C18 column (Phenomenex, Aschaffenburg, Germany)with 0.1% formic acid (eluent A) and 0.1% formic acid in acetonitrile (eluent B) as elution solvents. An injection volume of 1 µl and a flow rate of 0.4 mL/min was used. The gradient elution started at 23% B which was held for 3 min, afterward the concentration was increased to 30% B at 4 min, with another increase to 40%B at 6.5 min, at 7 min 100% B was used which was hold for 1 min, at 8.5 min the column was equilibrated at starting conditions. The column oven was set to 40°. Data acquisition and instrumental control were performed with Analyst 1.7 software (Sciex, Darmstadt, Germany). The data was analyzed with MultiQuant 3.0.3 (Sciex, Darmstadt, Germany) and Metaboanalyst. Features with more than 70% of missing values were removed. Missing values were replaced by LoDs (1/5 of the minimum positive value of each variable). Comparisons between two groups were performed with the Wilcoxon matched-pairs signed rank test (two-sided, 95% confidence level). Graphs and statistics were created in Prism v9 (GraphPad).

### 16S rRNA gene amplicon sequencing

Murine fecal samples were collected at indicated time points during the experiment and snap frozen in liquid nitrogen. After DNA isolation, targeted 16S rRNA gene amplicons were produced by fusing barcodes plus adapters using a 2-step PCR. For DNA isolation, the MaxWell RSC Faecal Microbiome DNA Kit (Promega) was used with bead beating. For the latter, the FastPrep-24 (MP Biomedicals Germany GmbH) was used, supplied with a CoolPrep adapter (MP Biomedicals, cooled with dry ice); thrice for 20 s of beating at a speed of 6 m/s, each followed by a 30 s break for cooling. Cycle times for the first and the second PCR had been adjusted for denaturation to 10 s, for annealing to 20 s and for extension to 45 s. After library preparation and equimolar pooling of the amplicons, the samples were sequenced using a MiSeq (Illumina). Raw sequencing data were analysed in a UPARSE-based pipeline clustering sequences down to zOTUs (denoised operational taxonomic units) with www.imngs2.org, which includes the Taxonomy-Informed Clustering (TIC) algorithm. Downstream analysis of zOTU tables was conducted using Rhea with default settings. Briefly, reads were normalized and spurious taxa were removed.

### Example 2: Results

The experiments were performed as described in Example 1.

**The microbial metabolite DAT modulates clinical and experimental GvHD and GvL**

Utilizing targeted mass spectrometry (MS), the inventors quantified fecal levels of DAT and ICA in 50 patients (patient characteristics in **Table 1)** before (day -7) and after allo-HSCT (obtained between days +7 to +21, **Fig. 1A****).** Both metabolites declined significantly after allo-HSCT **(****Fig. 1B****).** Based on established thresholds (DAT=0.005 µmol/g dry feces; ICA=0.035 µmol/g dry feces), the inventors categorized patients into high and low DAT or ICA groups **(Table 1).** High levels of DAT and ICA correlated with improved two-year overall survival **(****Fig. 1 C****).**

The inventors then explored whether DAT treatment could protect mice receiving murine allogeneic bone marrow transplantation (allo-BMT) from experimental GvHD induced by major histocompatibility complex (MHC) mismatch (C57BL/6 donors transplanted into BALB/c recipients). Recipients received donor bone marrow (BM) and T cells, while a control group received only donor BM. DAT was administered daily via oral gavage from day -7 before allo-BMT until day 0, and ICA was administered as described before (day -7 until day +12 after allo-BMT) **(****Fig. 1D****).** The T cell control group received oral gavage with the carrier only. Treatment with DAT or ICA significantly mitigated GvHD-induced weight loss and prolonged overall survival compared to carrier-treated mice **(****Fig. 1E****).**

Given that the therapeutic effect of allo-HSCT relies on anti-tumor responses exerted by donor T cells, the inventors further explored the impact of metabolites on GvL using the Ba/F3 FMS-like tyrosine kinase 3-internal tandem duplication-luciferase (Ba/F3 FLT3-ITD-Luc) acute lymphoid leukemia (ALL) model. BALB/c recipients were inoculated with syngeneic Ba/F3 leukemia cells and received donor BM. The inventors transplanted allogeneic T cells (C57BL/6 → BALB/c) or PBS two days post-inoculation. As before, mice were treated with metabolites or carrier alone **(****Fig. 1F**), and the ensuing Ba/F3 leukemia cell expansion was monitored via bioluminescence imaging (BLI, **Fig. 1** **G, H).** The inventors observed a significantly reduced tumor burden and prolonged survival in mice that received allogeneic T cells than in those that received BM only **(****Fig. 1I**). DAT treatment delayed tumor progression, resulting in moderately prolonged survival compared to mice transplanted with allogeneic T cells or BM only. Consistent with previous reports, ICA treatment did not negatively affect GvL responses **(****Fig. 1** **H, I**).

Given that DAT improved GvL responses in mice, the inventors assessed the incidence of relapse in the inventors' allo-HSCT patient cohort stratified according to high and low DAT levels, adjusting for transplantation-related mortality (TRM) as a competing risk. High DAT levels were independently associated with significantly lower relapse rates in patients receiving allo-HSCT **(****Fig. 1J****).** These results indicate that DAT may exert dual roles in allo-HSCT, protecting from GvHD while contributing to GvL.

The intestinal epithelium, located at the host-microbiome interface, plays a crucial role in GvHD-related morbidity when damaged. Preserving the integrity of the intestinal barrier has proven effective in mitigating GvHD. The inventors therefore next focused on DAT's impact on intestinal epithelium.

### DAT treatment promotes intestinal organoid growth and protects against toxicity in vitro

To model the impact of metabolite treatment on the intestinal barrier, the inventors administered DAT to murine small intestinal crypt-derived (SI) organoids **(****Fig. 2A****).** As before, the inventors used ICA as a positive control for a metabolite known to mitigate GvHD in an IFN-I-dependent manner. Metabolite stimulations were performed with the median stool metabolite concentration assessed in patients at day -7 before allo-HSCT (approximately 50 µM ICA and 100 µM DAT) **(****Fig. 1B****).**

DAT treatment of organoids significantly increased organoid size **(****Fig. 2B****)** and the abundance of Lgr₅-GFP^{high} intestinal stem cells (ISCs) **(****Fig. 2C****)** as a surrogate for increased proliferation. Consistently, DAT treatment enhanced organoid expansion, indicated by increased numbers of organoids that were established after passage compared to untreated organoids **(****Fig. 2D**). In contrast, ICA treatment exhibited no effect on the size, ISC abundance or organoid numbers **(****Fig. 2B-D****).**

To examine whether DAT could protect the intestinal epithelium, the inventors challenged murine organoids with different toxic regimens relevant in patients receiving allo-HSCT in the presence or absence of metabolites.

T-cell toxicity is mediated by inflammatory cytokines such as IFNγ. To mimic the toxic effect of these soluble mediators on the epithelium, the inventors co-cultured C57BL/6 SI organoids with CD3/28-bead activated allogeneic CD3+ T cells (MACS isolated from BALB/c spleens) suspended in the surrounding media but without direct contact between them. As a result, the expansion of organoids after passaging was reduced compared to control organoids cultured without T cells. Treatment with DAT rescued organoid regeneration, while ICA was less effective **(****Fig. 2E****).**

Donor CD8+ T cells can infiltrate the recipient intestinal epithelium and target epithelial cells in a direct, TCR-MHC-mediated allo-reaction. To model this interaction, the inventors co-cultured intestinal organoids with allogeneic intraepithelial T cells for 48 hours in direct contact and assessed cell death in organoids by propidium iodide staining (normalized for cell count using Hoechst staining, **Fig. 2F****, right).** In this model, treatment with ICA, but not DAT, protected organoids from direct, T cell-mediated cytotoxicity **(****Fig. 2F****, left).** This may be explained in part by ICA-induced downregulation of MHC class II expression in organoids **(****Fig. 9A****).** To mimic conditioning damage, the inventors exposed organoids to irradiation alone or in presence of metabolites. The inventors observed that treatment with DAT but not ICA improved the number of viable organoids and organoid expansion following irradiation **(****Fig. 9B****).**

These findings suggest that DAT promotes regeneration of the intestinal epithelium *in vitro* and protects it from radiation and inflammatory cytokines without affecting the direct interaction between T cells and epithelial cells.

### DAT treatment protects the intestinal barrier and stem cell compartment from tissue damage in vivo

To investigate the impact of metabolites on the intestinal epithelium in *vivo,* the inventors administered DAT and ICA to mice and assessed barrier damage and the regenerative capacity of the intestinal stem cell compartment after allo-BMT **(****Fig. 2G****).**

Mice received total-body irradiation (TBI) and the influx of CD11b+Ly6G+ granulocytes to the intestinal lamina propria was assessed at day +3 as a surrogate of intestinal barrier damage. Metabolite treatment reduced granulocyte influx compared to carrier-treated mice **(****Fig. 2H****).** To assess the regenerative capacity of the ISC compartment, the inventors developed an organoid-based assay in which intestinal crypts were isolated from the small intestine on day +7 after allo-BMT, and a defined crypt number was seeded into organoid cultures. After 72 hours, organoid numbers were counted. A higher number of organoids established is suggestive of more intact ISCs. Transplantation of allo-BM and T cells resulted in reduced SI organoid regeneration compared to allo-BM alone **(****Fig. 2I**). However, treatment with DAT or ICA in mice that received BM and allogeneic T cells restored organoid regeneration to levels comparable with BM alone **(****Fig. 2I**). The inventors confirmed this protective effect in organoids derived from large intestinal (LI) crypts **(****Fig. 9C****).** Similar results for barrier integrity and SI organoid recovery were obtained when intestinal damage was induced by TBI and syngeneic BMT and the anthracycline doxorubicin **(****Fig. 9D-E****)**.

Histological analysis of the large intestine revealed a reduction in GvHD histopathological scoring **(****Fig. 2J****)** and T cell infiltration **(****Fig. 2K****)** as well as preservation of the mucus layer **(****Fig. 2L****)** in DAT or ICA-treated mice compared to controls. Systemically, metabolite treatment significantly reduced serum IFNγ concentrations at day +7 after allo-BMT. In addition, DAT treatment resulted in a trend towards reduced TNFα and elevated IL-10 concentrations in serum **(****Fig. 9F****).**

In summary, this data indicates that DAT exerts its protective effect in *vivo* by protecting the intestinal epithelium.

### DAT treatment remains effective despite the use of broad-spectrum antibiotics

The interplay of the bacteriome, fungome, and virome correlates with metabolite expression, while conversely, metabolite treatment itself may shape intestinal microbial communities. Therefore, the inventors assessed whether metabolite treatment affected the murine intestinal microbiome. Daily metabolite treatment in mice via oral gavage for seven days did not significantly alter the family-level taxonomic composition compared to carrier treatment **(****Fig. 3A**). Similarly, neither alpha diversity nor beta diversity differed significantly between metabolite- or control-treated mice.

To investigate whether the protective effect of DAT required an intact microbiome, the inventors treated germ-free (GF) mice with bacterial metabolites, performed allo-BMT, and assessed organoid regeneration as before **(****Fig. 3B**). The efficacy of metabolites was strongly reduced in GF mice: DAT treatment resulted in a non-significant trend towards improved organoid recovery, while ICA had no effect **(****Fig. 3B**).

Since epithelial and immune functions are altered in GF mice, the inventors assessed whether colonization with microbiota may be required for them to respond to metabolite treatment. Therefore, the inventors isolated intestinal crypts from GF mice that received fecal microbiota transplantation (FMT) from specific-pathogen-free (SPF) donor mice seven days earlier or a mock-FMT and stimulated isolated crypts *ex vivo* with metabolites directly after isolation. SI crypts were responsive to DAT treatment, whether they were isolated from FMT-recipients or GF mice **(****Fig. 3C****).** This suggested that the poor efficacy of metabolites in GF mice may be due to an inherent lack of microbial signals (e.g., microbe-associated molecular patterns) or impaired intestinal development.

To overcome this limitation, the inventors performed gut decontamination in SPF mice using an antibiotic cocktail consisting of ampicillin and enrofloxacin administered via drinking water for seven days before initiating metabolite treatment. This resulted in the complete suppression of culturable bacteria and no amplification of 16S rRNA bacterial reads. In this setting, the protective effect of DAT in mice receiving allo-BMT was restored **(****Fig. 3D****).**

Since gut decontamination in patients receiving allo-HSCT was ineffective in reducing mortality, it is no longer the standard of care. However, the use of broad-spectrum antibiotics remains widespread in these patients and is associated with dysbiosis and GvHD-related microbial signatures. To test whether metabolite treatment remains effective during therapy with broad-spectrum antibiotics, the inventors administered meropenem to mice for seven days before initiating metabolite treatment and performing allo-BMT. Administration of meropenem aggravates colonic GvHD. Consistently, meropenem significantly hampered organoid regeneration, reducing LI crypt-derived intestinal organoids compared to mice that did not receive antibiotics. However, metabolite treatment with DAT or ICA restored organoid recovery, compensating for antibiotic-induced dysbiosis **(****Fig. 3E**).

Fecal microbiota transplantation (FMT) is approved in antibiotics-refractory *C. diff.* colitis and in clinical trials for gastro-intestinal GvHD. Although the exact mechanisms are not fully understood, mitigating the adverse effects of broad-spectrum antibiotics and restoring microbial diversity may be important. Given that that exogenous administration of chemically synthesized DAT could enhance intestinal regeneration despite dysbiosis, the inventors investigated whether a "precision" FMT using a flavonoid-degrading, DAT-producing bacterial probiotic as a physiological source of DAT might be equally effective. After exposure to ampicillin and enrofloxacin, mice received suspensions of *Flavonifractor plautii* (*F. plautii*) strains isolated from human (DSM 6740) or murine (DSM 26117) feces or control FMT with cecal content from SPF mice followed by allo-BMT. Human-isolated *F. plautii* administration significantly improved organoid regeneration on day seven after allo-BMT compared to control FMT **(****Fig. 3F****).**

These findings suggest that metabolite-based treatments remain effective even after exposure to broad-spectrum antibiotics and that intestinal regeneration can be enhanced through synthetic compounds or flavonoid-degrading bacteria.

### DAT-induced organoid regeneration requires intact STING but not IFN-I signaling.

Given the link between DAT and IFN-I responses, the inventors next investigated whether intact IFN-I signaling or IFN-I-inducing pathways were required for DAT's protective effect on the intestinal epithelium. To this end, the inventors utilized intestinal organoids deficient for the IFN-I receptor (IFNaR^{-/-}), the stimulator of interferon genes (STING Goldenticket, STING^{GT/GT}), or the mitochondrial antiviral-signaling protein (MAVS^{-/-}).

The improved growth of DAT-treated SI organoids was abrogated in the absence of STING signaling **(****Fig. 4A****, left).** In contrast, DAT's effect did not depend on intact IFNaR **(****Fig. 4A****, right)** nor MAVS signaling. Similarly, in the absence of functional STING, DAT treatment did not significantly increase organoid size **(****Fig. 4B**). Like in wild-type (WT) organoids, ICA treatment did not affect IFNaR^{-/-}, STING^{GT/GT}, or MAVS^{-/-} organoid growth.

To examine whether STING signaling was required for DAT's ability to protect against inflammatory cytokines, the inventors co-cultured STING-deficient organoids with WT allogeneic T cells in the surrounding media (without direct contact between organoid and T cells, as in **Fig. 2E**). The protective effect of DAT was abrogated in the absence of epithelial STING signaling **(****Fig. 4C****).**

To confirm these findings in *vivo,* the inventors performed metabolite treatment in IFNaR^{-/-}or STING^{GT}/^{GT} mice (C57BL/6 background), followed by allo-BMT from WT BALB/c donors. The inventors observed that DAT-induced organoid regeneration was abrogated in STING^{GT}/^{GT} recipients but remained intact in recipient mice lacking IFNaR **(****Fig. 4D****).** In contrast to previous reports, the protective effect of ICA on the intestinal stem cell compartment was not dependent on IFN-I and remained intact in both knockout models.

### DAT promotes the expansion of metabolically-active intestinal stem cells

To better understand DAT's effect on intestinal organoids and the role of STING in this context, the inventors conducted single-cell RNA sequencing (scRNA-seq) of WT and STING-deficient organoids after treatment with DAT or control **(****Fig. 4E****).** The inventors annotated cells via a combination of graph-based clustering, evaluation of marker signature expression, and automated cell type annotation based on a scRNA-seq reference dataset from the murine intestine (see *Methods* for a detailed description of the approach). This enabled us to identify all major intestinal epithelial cell types **(****Fig. 4F****)**.

Using this approach, cells with stem cell characteristics, namely ISCs and transit-amplifying cells (TA), resided on a continuum and were poorly separated. To resolve this continuum and enhance the precision of cell type annotation, the inventors conducted trajectory analysis on the subset of cells initially classified as ISC or TA. Cells were ordered in pseudotime according to their expression of genes that differentiate ISC and TA populations in the reference dataset. Based on trajectory states in pseudotime and their expression of stem cell markers, the inventors categorized these stem-like cells into three groups: ISC, ISC II, and TA **(****Fig. 4G****,** detailed description of the approach and the branching of ISC II and TA on component 2 in the methods example). Here, ISC and TA correspond to the classical definition of ISCs and TA cells, while ISC IIs represent an intermediate population.

Next, the inventors examined whether metabolite treatment altered the abundance of these cell types. In WT organoids, treatment with DAT led to an expansion of ISC-II. This effect was attenuated in STING-deficient organoids. In direct comparison, the abundance of ISC-II was reduced in DAT-treated STING-deficient (Goldenticket (GT) DAT) versus DAT-treated WT organoids (WT DAT). Conversely, the inventors observed an increased abundance of differentiated enterocytes in GT DAT vs WT DAT **(****Fig. 4H****).**

Considering that DAT strongly affected ISC-IIs, the inventors focused on them for detailed analysis. According to gene expression analysis and gene set enrichment analysis (GSEA), ISC-IIs were classified as an ISC subpopulation with a high expression of the intestinal stem cell markers Lgr₅ and Olfm4 accompanied by upregulation of pathways indicating a high metabolic and proliferative activity.

### STING regulates DAT-activated mTORC1 signaling and cellular stress in intestinal stem cells

In steady-state organoids, the transition from ISC to ISC-II was associated with increased mTORC1 signaling, a well-established regulator of cellular metabolism and proliferation (ISC-II vs. ISC). Further upregulation of mTORC1 signaling led to the transition of ISC-II to the more differentiated TA cells (TA vs. ISC-II). The metabolic activity in ISC-IIs was partly due to increased oxidative phosphorylation (OXPHOS), which is required for energy production, but also generates reactive oxygen species (ROS), damaging mitochondria and releasing mitochondrial DNA (mtDNA) in the process. Consequently, the inventors observed an upregulation of cellular stress pathways, particularly related to ROS, and pathways for organelle maintenance and self-renewal through autophagy, specifically mitophagy (i.e., the selective degradation of mitochondria by autophagy) during the transition from ISC through ISC-II to TA.

Given that DAT promoted ISC-II abundance and that mTORC1 signaling is crucial for the ISC to ISC-II transition, without wishing to be bound by any theory, the inventors hypothesized that DAT may act via mTORC1. To test for this, the inventors treated SI organoids with the mTOR inhibitor rapamycin. Rapamycin abrogated DAT-induced organoid growth **(****Fig. 5A**), and inhibition of the mitogen-activated protein (MAP) kinase pathway, an upstream activator of mTORC1, with U0126, had the same effect. Consistently, rapamycin compromised the expansion of Lgr₅^{high} ISCs in response to DAT **(****Fig. 5B****).**

The transition of intestinal stem cells to ISC-IIs required activation of mTORC1 signaling following DAT treatment. Gene Set Enrichment Analysis (GSEA) of DAT-treated versus untreated WT organoids revealed that mTORC1 signaling and cellular stress responses were reduced in ISC, while OXPHOS was downregulated in ISC-II compared to untreated WT organoids. In contrast, GSEA of DAT-treated versus untreated STING-deficient organoids showed persistent upregulation of mTORC1 signaling in both ISC and ISC-II, without any downregulation of metabolic activity or cellular stress responses **(****Fig. 5C****).**

Without wishing to be bound by any theory, the inventors hypothesized that STING may attenuate cellular stress by counterbalancing mTORC1 signaling and metabolic activation in ISCs. STING-dependent self-renewal by autophagy can be triggered by cytosolic mitochondrial DNA (mtDNA)-sensing. OXPHOS activation and ROS production have been attributed to mitochondrial DNA (mtDNA) release into the cytosol following metabolic activation. To test if autophagy is required for DAT's beneficial effect, the inventors inhibited autophagy using chloroquine, which effectively abolished DAT-induced organoid growth **(****Fig. 5D**) and decreased the abundance of Lgr₅^{high} ISCs in response to DAT. Likewise, direct inhibition of mTOR (with rapamycin) or STING (with H-151, a small-molecule STING inhibitor), resulted in a decrease of Lgr₅^{high} ISCs **(****Fig. 5B**).

To test whether the compromised downregulation of OXPHOS observed in DAT-treated, STING-deficient organoids contributed to the loss of DAT's growth-promoting effect was related to ROS, the inventors treated STING-deficient organoids with the antioxidant N-acetylcysteine (NAc). Reducing ROS levels restored their responsiveness to DAT treatment **(****Fig. 5E****).**

ISC proliferation is crucial for epithelial regeneration following tissue damage. Within intestinal crypts, epidermal growth factor (EGF) is a major regulator of proliferation. EGF drives regeneration upon irradiation, and mTORC1 agonists can support this. Given that DAT promotes ISC proliferation via mTORC1 signaling, the inventors investigated whether DAT treatment could compensate for the absence of EGF. Therefore, the inventors evaluated the growth-promoting effect of DAT under normal growth conditions in ENR media (containing EGF, Noggin, and R-spondin) **(****Fig. 2D**) or EGF-depleted NR media. DAT-induced organoid growth was significantly enhanced in EGF-reduced culture conditions **(****Fig. 5F**). Following additional depletion of endogenous EGF (which can be produced by the organoids themselves) by anti-EGF antibodies, DAT exhibited only minor compensatory capacity.

Inflammation can induce ISC exhaustion and their transition into non-functional Paneth cells, characterized by aberrant *Lgr₅* localization to the "upper crypt" (above +4 position) of intestinal crypts. Since allo-BMT is marked by severe intestinal inflammation, the inventors investigated if aberrant *Lgr₅* occurred in the upper crypt and whether DAT could promote the retention of Lgr₅+ stem cells in the niche. The inventors conducted *Lgr₅* in *situ* hybridization in large intestines on day seven after allo-BMT and assessed the distribution of *Lgr₅* expression in intestinal crypts. Aberrant *Lgr₅* signal was detectable in most crypts of carrier-treated mice **(****Fig. 5G****).** However, in DAT-treated mice, aberrant *Lgr₅* expression was significantly reduced. In STING-deficient mice, the inventors observed no effect of DAT on the localization of *Lgr₅* expression.

In summary, DAT-induced mTORC1 signaling is crucial for the expansion of ISC-II, and a functional cellular stress response mediated by STING is required for organoid regeneration.

### DAT promotes growth in healthy donor and patient-derived human organoids

To validate the efficacy of DAT treatment in humans **(****Fig. 6A****),** the inventors treated LI organoids from healthy volunteers. Like in murine organoids, DAT treatment promoted organoid growth **(****Fig. 6B****).** The inventors next wondered if the efficacy on organoid growth of DAT was compromised in patients receiving allo-HSCT. Utilizing patient-derived organoids (PDOs) derived from intestinal biopsies of either SI or LI of allo-HSCT patients, the inventors found that DAT's growth- and size-promoting effect remained intact **(****Fig. 6C-D****).**

To confirm that the impact of DAT required intact STING signaling in humans, the inventors inhibited STING in SI and LI PDO via H151, followed by DAT treatment. H151-treated PDOs grew poorly compared to control organoids, suggesting a requirement for STING in organoid growth. Like in murine STING^{GT/GT} organoids, DAT treatment was ineffective after H151 inhibition **(****Fig. 6B****).**

The inventors confirmed the protective effect of DAT in indirect organoid/T cell co-cultures in both SI and LI PDOs **(****Fig. 6E****).** This effect was present despite elevated IFNγ concentrations in DAT-treated co-cultures **(****Fig. 6F****).**

These data conclude that DAT exerts STING-dependent protective effects in the intestinal epithelium of healthy volunteers and remains effective in patients receiving allo-HSCT.

### DAT treatment only moderately impacts T cell activation

Besides intestinal barrier protection, mechanisms that may contribute to DAT's effect include (i) increased frequency of donor regulatory T cells (Tregs), (ii) impaired function of tissue-damaging alloreactive CD8+ effector cells, or (iii) reduced capacity of host antigen presenting cells (APCs) to promote T cell alloreactivity. Given that the inventors found elevated IFNγ levels in DAT-treated human organoid/T cell co-cultures **(****Fig. 6F****),** the inventors stimulated CD3+ T cells from peripheral blood mononuclear cells (PBMC) of healthy volunteers with anti-CD3/CD28 beads for 24 hours, and added metabolites in escalating doses **(****Fig. 7A**).

Direct treatment with the metabolites DAT or ICA resulted in opposing effects: while the ratio of CD4+ to CD8+ T cells remained unaffected by either metabolite, DAT, but not ICA treatment, reduced the frequency of Tregs in a dose-dependent manner **(****Fig. 7B**). The frequency of CD4+ T cells expressing the inflammatory cytokine interferon y (IFNγ) was not affected by either metabolite. Only after applying unphysiologically-high DAT doses (1000 µM, henceforth referred to as "high-dose DAT"), did the inventors observe increased IFNγ expression per cell by mean fluorescence intensity (MFI). ICA had the opposite effect and suppressed IFNγ expression **(****Fig. 7C****).** Consistent with increased activation, high-dose DAT promoted the immune checkpoint receptor PD-1 expression on CD4+ T cells.

On CD8+ T cells, neither low nor high-dose DAT treatment increased the abundance of IFNγ producing cells or the expression levels of IFNγ or PD-1 **(****Fig. 7D**). Instead, the inventors observed reduced IFNγ expression following low-dose DAT treatment **(****Fig. 7D**). ICA treatment lowered the abundance of IFNγ+ CD8+ cells and the expression of IFNγ and PD-1 **(****Fig. 7D**) in a dose-dependent manner. These results indicate that only high-dose DAT can promote an effector phenotype in human T cells. This was corroborated by comparing the ratio of regulatory to effector T cells: high-dose DAT resulted in significantly reduced ratios of Tregs to effector CD4+ **(****Fig. 7E**) and CD8+ **(****Fig. 7F**) T cells. The inventors observed a similar reduction in Treg to effector T cell ratios in DAT-treated murine splenic CD4+ and CD8+ T cells. As the GvL effect is mediated in part by T cell cytotoxicity, the inventors next assessed effector CD8+ T cell function by quantifying Granzyme B expression. High-dose DAT treatment increased the abundance of Granzyme B+ CD8+ T cells and Granzyme B expression. Conversely, ICA had the opposite effect **(****Fig. 7G-H****)**, suppressing cytotoxic protein expression.

The inventors then utilized the murine allo-BMT model **(****Fig. 1D****)** to assess the impact of low-dose DAT or ICA on murine donor T cells in *vivo.* To this end, the inventors analyzed donor (H-2K^{b+}) T cells isolated from the intestinal epithelium at day +7 after allo-BMT. Metabolite treatment did not affect epithelial donor CD4+ T cells and the inventors did not observe any effects on Treg abundance following DAT or ICA. However, DAT treatment resulted in a moderately increased abundance of IFNγ+ CD8+ T cells and expression of PD-1 **(****Fig. 7K**) and aligns with a previous report showing that DAT can directly activate murine TCR-stimulated CD8+ T cells *in vitro.* In contrast, ICA treatment trended towards a reduced frequency of IFNy+CD8+ T cells and elevated PD-1 expression.

Finally, the inventors could not detect any significant effects following stimulation with low-dose DAT on human or murine APCs.

The inventors' data indicate that DAT at physiological doses (i.e., low-dose, corresponding to fecal levels in patients receiving allo-HSCT) only moderately impacted T cells. Therefore, its beneficial effect on GvHD is unlikely to be mediated by Treg promotion, suppression of allogeneic T cells, or APC priming of alloreactivity. In the context of GvHD, DAT's T-cell-dependent effect appeared to be secondary to its robust effect on epithelial cells, while the dose-dependent amplification of both human and murine CD8+ T cells may partially explain DAT's impact on GvL **(****Fig. 1I**).

### Example 3: Discussion

The experiments were performed as described in Example 1 and the results thereof as described in Example 2.

Herein, the inventors demonstrate that the microbial metabolite DAT, identified in patients receiving allo-HSCT and associated with improved overall survival and reduced relapse at two-year follow-up, concurrently mitigates GvHD and contributes to anti-tumor immune responses in murine in *vivo* and human *ex vivo* models. This "dual function" of DAT is unique among other microbial metabolites in allo-HSCT and arises from DAT's activating effects on T cells and its protective role in maintaining the intestinal barrier (summarized in **Fig. 8****, upper panel):**

There is robust evidence that the intestinal microbiome impacts the outcome following allo-HSCT and the incidence of GvHD. The perturbation of the microbiome due to antibiotic use and the adverse consequences of the ensuing dysbiosis are well documented. Consequently, efforts are underway to restore microbial diversity after allo-HSCT, i.e., via FMT, with promising outcomes. Similarly, microbiota-derived metabolites are associated with positive outcomes following allo-HSCT. Specific metabolites, such as SCFAs (e.g., butyric acid), bile acids, and tryptophan-derived metabolites like ICA, shape the intestinal immune cell compartment towards an anti-inflammatory environment by promoting the expansion of Tregs, releasing anti-inflammatory and regenerative cytokines such as IL-10 and IL-22, and suppressing effector T cells and APCs.

Consistently the inventors observed that ICA prevented T-cell toxicity in the co-culture model of *in vitro* GvHD. The inventors attribute this effect to an immunosuppressive role of ICA in allo-BMT, evidenced by reduced T cell activation and downregulation of MHC II expression in epithelial cells and monocytes.

In contrast, DAT enhanced effector T cell phenotypes in vivo and in vitro, especially at high doses. DAT protected from GvHD and produced only a moderate increase in murine CD8+ T cells in *vivo.* This may partly explain the capacity of DAT to significantly delay Ba/F3 FLT3-ITD-Luc acute leukemia progression without compromising GvHD protection. DAT had only minor effects on human APCs, aligning with previous reports that DAT promotes APC activation only in combination with viral co-stimulation but not on its own.

Compared to the moderate effects of DAT on immune cells, DAT was very potent in stimulating intestinal epithelial cells. DAT increased the epithelial turnover in human and murine organoids, expanded the ISC compartment, and improved resistance to irradiation and cytokine-mediated toxicity. *In vivo,* DAT protected the intestinal barrier, as assessed by granulocyte influx, and enhanced intestinal regeneration, as evaluated by the organoid regeneration assay. Protecting the intestinal barrier from conditioning chemo- or radiotherapy and T cell toxicity is an effective mechanism to prevent early allogeneic T cell priming and subsequent GvHD. Additionally, DAT prevented the thinning of the intestinal mucus layer after allo-BMT, which has been associated with aggravated GvHD. The inventors' data, therefore, indicate that at physiological doses (e.g. doses < 275 mg/kg, preferably < 200 mg/kg, such as a dose in a range of from 1 to 100 mg/kg), DAT's robust protective effect on the intestinal epithelium is balanced with moderate T cell activation, resulting in a benefit for both GvHD and GvL.

The inventors also aimed to assess whether DAT treatment remained effective without an intact microbiome or during antibiotics-induced dysbiosis. DAT's protective effect on ISCs was compromised in GF mice in *vivo.* In contrast, *ex vivo,* DAT treatment was effective in both GF and FMT-colonized GF mice-derived crypts, consistent with data showing that organoids maintain a robust transcriptome/proteome phenotype independent of GF status. The inventors conclude that the in *vivo* effect of DAT treatment is relying on additional microbial signals (e.g., microbe-associated molecular patterns) and physiological intestinal development, which are compromised in GF mice. Along these lines, the ability of DAT to promote organoid regeneration was restored following gut decontamination with ampicillin/enrofloxacin in SPF mice.

Intestinal dysbiosis is common in patients receiving allo-HSCT and is associated with GvHD and broad-spectrum antibiotics, such as meropenem. Despite meropenem treatment, both DAT and ICA still induced tissue regeneration in the mouse allo-BMT model. Previously, the inventors showed that broad-spectrum antibiotics worsen the efficacy of immune-checkpoint-blockade in murine solid tumor models, but DAT treatment could restore efficacy. Together, these data suggest that endogenous DAT, produced by anaerobic microbiota such as *F. plautii* - identified in murine and allo-HSCT patient studies, may contribute to steady-state tissue regeneration and anti-tumor immunity.

FMT is the most advanced among the different microbiome-based therapies currently under development, including for the treatment of acute, steroid-refractory GvHD. Intriguingly, FMT of the flavonoid-degrading, DAT-producing human-isolated *F. plautii* significantly improved organoid regeneration following allo-BMT and broad-spectrum antibiotics-induced dysbiosis. Thus, a "precision" FMT using defined metabolite-mixtures or metabolite-producing bacterial strains may be a potent alternative to "pure" metabolites, able to supply DAT endogenously at the interface between microbiota and host epithelium.

The inventors' study finds that DAT-induced intestinal organoid growth is dependent on mTORC1 signaling. mTORC1 plays an essential role in intestinal homeostasis and regeneration and regulates metabolic activation and proliferation in response to growth factors like EGF. Its transient activation in ISCs after damage is crucial for regeneration and beneficial effects associated with fasting. Consistently, the inventors observed that DAT treatment in WT organoids expanded ISCs with elevated metabolic and proliferative capacity while retaining expression of the stem cell markers Lgr₅ and Olfm4 (ISC-II).

The STING pathway, known for its role in innate immunity, recognizes microbial and non-microbial ligands like cyclic di-nucleotides and free double-stranded (ds) DNA. These ligands, derived from intestinal microbiota or other sources, are constantly sensed by STING and induce tonic signaling and IFN-I responses, influencing intestinal homeostasis and local and systemic immunity. Synthetic direct cGAS / STING agonists like dsDNA can stimulate IFN-I responses to protect the intestinal epithelium and against GvHD in mice, even though the cellular targets, its associated mechanisms and the relevance in humans remain unclear. Since the DAT phenotype can be restored in STING deficient organoids by inhibiting ROS, the inventors' data suggest that DAT does not directly activate STING in ISCs. Instead, it utilizes STING for metabolic regulation following mTORC1 activation thereby maintaining an undifferentiated, regenerative stem cell state. Consequently, in STING deficient organoids, DAT-induced mTORC1 activation was dysregulated, and constitutively active during the transition from ISC to ISC-II. Dysregulated mTORC1 activation has been associated with depletion of the ISC pool as well as stem cell exhaustion following repetitive stress and aging, stem cell differentiation, and intestinal barrier damage induced by a Western diet. Furthermore, STING's regulatory function occurs independently of IFN-I signaling, consistent with previous reports in other cell types showing that STING can recognize the release of mtDNA following stress, thus contributing to metabolic regulation, and reports of elevated STING expression in ISCs. The inventors therefore propose the following model (summarized in **Fig. 8****, lower panel):** In WT organoids, DAT promotes ISC proliferation and metabolic activation by activating mTORC1 signaling and OXPHOS. ROS, a byproduct of OXPHOS, triggers mitochondrial stress and STING activation. Upon activation, STING provides a negative feedback loop that attenuates metabolic stress by regulating mTORC1 and promoting autophagy, increasing the ISC-II pool and limiting their further differentiation. As a result, DAT promotes a regenerative phenotype in organoids **(****Fig. 8****, lower panel (left)).** In contrast, STING deficiency results in a compromised ability to react to metabolic stress, unchecked mTORC1 signaling and ROS production. Consequently, ISCs undergo differentiation, and the capacity for organoid regeneration is abolished **(****Fig. 8****, lower panel (right)).**

Taken together, the inventors identify a microbial metabolite (DAT) with hitherto unknown beneficial, dual functions on GvHD and GVL in humans and mice and uncover an unexpected link between microbial metabolites, mTORC1 activation, and the STING pathway to induce regulation of the ISC compartment.

### Translational relevance

Restoring intestinal DAT levels, e.g., by exogenous administration of "pure" metabolites, flavonoid-degrading probiotics (e.g., *Flavonifractor plautii*)*,* or "precision" FMT products with defined metabolite composition and prespecified dose ranges, represents an attractive treatment approach during the use of broad-spectrum antibiotics in patients receiving allo-HSCT. Combining DAT with other metabolites may also provide synergisms for tailoring these therapies according to specific indications, i.e., regeneration-promoting metabolites for GvHD, immunity-enhancing metabolites for GVL and perhaps other cancer immunotherapies (e.g., chimeric antigen receptor (CAR) T cells, immune checkpoint inhibitors, bispecific antibodies). Finally, DAT's association with beneficial GvHD and GvL responses may be useful as a clinical biomarker for predicting clinical outcomes following allo-HSCT.

The features of the present invention disclosed in the specification, the claims, and/or in the accompanying figures may, both separately and in any combination thereof, be material for realizing the invention in various forms thereof.

## Claims

1. Desaminotyrosine (DAT) for use in preventing or treating graft-versus-host disease (GvHD) in a patient; preferably in preventing or treating a GvHD in a patient receiving an allogeneic stem cell transplantation.

2. DAT for use as an adjuvant in an immunotherapy, preferably cancer immunotherapy, of a patient; wherein, optionally, said immunotherapy, preferably cancer immunotherapy, comprises an allogeneic stem cell transplantation, a CAR T cell therapy, a T cell receptor therapy, and/or an immune checkpoint inhibiting treatment.

3. DAT for use according to claim 1 or 2, wherein DAT is administered to said patient in combination with at least one further microbial metabolite; wherein, optionally, said at least one further microbial metabolite is selected from indole-3-carboxaldehyde, butyric acid, and a combination thereof.

4. DAT for use according to any one of the foregoing claims, wherein DAT is administered to said patient by administering a DAT-producing microorganism to said patient;
preferably a DAT-producing microorganism selected from *Acinetobacter* sp., *Bacteroides* sp., *Bifidobacterium* sp., *Clostridium* sp., *Enterococcus* sp., *Escherichia* sp., *Eubacterium* sp., *Flavonifractor* sp., *Klebsiella* sp., *Lactiplantibacillus* sp., *Lactobacillus* sp., *Pseudomonas* sp., and *Staphylococcus* sp., such as a DAT-producing microorganism selected from *Acinetobacter baumannii, Bacteroides distasonis, Bacteroides fragilis, Bacteroides thetaiotaomicron, Bifidobacterium Bifidum, Clostridium botulinium, Clostridium sporogenes, Enterococcus faecalis, Enterococcus faecium, Escherichia coli, Eubacterium lentum, Eubacterium ramulus, Flavonifractor plautii, Lactiplantibacillus pentosus, Lactobacillus fermentum,* and *Pseudomonas aeruginosa;*
more preferably *Flavonifractor plautii;*
even more preferably *Flavonifractor plautii* DSM 6740 or *Flavonifractor plautii* DSM 26117.

5. DAT for use according to any one of the foregoing claims, wherein DAT is administered in combination with at least one microorganism;
preferably in combination with at least one microorganism selected from *Acinetobacter* sp., *Akkermansia* sp., *Bacteroides* sp., *Bifidobacterium* sp., *Clostridium* sp., *Enterococcus* sp., *Escherichia* sp., *Eubacterium* sp., *Faecalibacterium* sp., *Flavonifractor* sp., *Klebsiella* sp., *Lactiplantibacillus* sp., *Lactobacillus* sp., *Pseudomonas* sp., *Staphylococcus* sp., and combinations thereof, such as in combination with at least one microorganism selected from *Acinetobacter baumannii, Akkermansia muciniphila, Bacteroides distasonis, Bacteroides fragilis, Bacteroides thetaiotaomicron, Bifidobacterium Bifidum, Clostridium botulinium, Clostridium innocuum, Clostridium sporogenes, Enterococcus faecalis, Enterococcus faecium, Escherichia coli, Eubacterium lentum, Eubacterium ramulus, Flavonifractor plautii, Lactiplantibacillus pentosus, Lactobacillus fermentum, Pseudomonas aeruginosa,* and combinations thereof;
more preferably in combination with *Flavonifractor plautii;*
even more preferably in combination with *Flavonifractor plautii* DSM 6740 or *Flavonifractor plautii* DSM 26117;
wherein, preferably, said at least one microorganism comprises at least one microorganism producing DAT and optionally producing at least one further microbial metabolite.

6. DAT for use according to any one of the foregoing claims, wherein DAT is administered to said patient in the form of a powder, a solution, a suspension, an emulsion, a capsule, a tablet, and/or granules; and/or
wherein DAT is administered to said patient in the form of a stool transplant.

7. DAT for use according to any one of the foregoing claims, wherein said patient is **characterized by** a stool comprising an amount of 0.005 µMol DAT per gram of dried stool or less; and/or
wherein said patient is **characterized by** a stool which does not comprise *Flavonifractor* sp., *Lachnospiraceae* sp., and/or *Oscillospiraceae* sp., preferably which does not comprise *Flavonifractor plautii.*

8. DAT for use according to any one of the foregoing claims, wherein said patient is a patient receiving an antibiotic treatment.

9. DAT for use according to any one of the foregoing claims, wherein said patient is **characterized by** an intact STING1 gene and/or a normal signaling of a stimulator of interferon genes (STING) pathway.

10. DAT for use according to any one of the foregoing claims, wherein DAT is used for promoting a regeneration of an intestinal epithelium and/or for an anti-tumor activity by activating effector T cells, optionally by reducing a ratio of Tregs to effector T cells.

11. DAT for use according to any one of the foregoing claims, wherein DAT is administered to said patient in a dose in a range of from 1 to 100 mg/kg, preferably in a dose in a range of from 5 to 50 mg/kg, more preferably in a dose in a range of from 10 to 20 mg/kg.

12. A method for predicting an outcome of an immunotherapy, preferably cancer immunotherapy, comprising the steps of
a) providing a sample of a patient, preferably a stool sample,
b) determining a level of DAT in said sample and comparing said level of DAT to a control;
wherein an increased level of DAT in said sample of said patient compared to said control is indicative for an improved overall survival and/or a reduced relapse; and/or
wherein a decreased level of DAT in said sample of said patient compared to said control is indicative for a reduced overall survival and/or an increased relapse.

13. The method according to claim 12, wherein step b) comprises determining whether said sample of said patient comprises more than 0.005 µMol DAT per gram of dried stool, preferably more than 0.02 µMol DAT per gram of dried stool, more preferably more than 0.12 µMolDAT per gram of dried stool; wherein a presence of more than 0.005 µMol DAT per gram of dried stool, preferably more than 0.02 µMol DAT per gram of dried stool, more preferably more than 0.12 µMolDAT per gram of dried stool in said sample is indicative for an improved overall survival and/or a reduced relapse.

14. The method according to claim 12 or 13, wherein step b) comprises determining whether said sample of said patient comprises 0.01 µMolDAT per gram of dried stool or less, preferably 0.005 µMol DAT per gram of dried stool or less, more preferably less than 0.005 µMol DAT per gram of dried stool, even more preferably 0.0 µMol DAT per gram of dried stool; wherein a presence of 0.01 µMol DAT per gram of dried stool or less, preferably 0.005 µMol DAT per gram of dried stool or less, more preferably less than 0.005 µMol DAT per gram of dried stool, even more preferably 0.0 µMol DAT per gram of dried stool in said sample is indicative for a reduced overall survival and/or an increased relapse.

15. The method according to any one of claims 12-14, wherein said immunotherapy comprises an allogeneic stem cell transplantation, a CAR T cell therapy, a T cell receptor therapy, and/or an immune checkpoint inhibiting treatment.
